# EUROPEAN PATENT SPECIFICATION

(11) **EP 3 927 364 B1**
(45) Date of publication and mention of the grant of the patent: **15.04.2026**
(21) Application number: 20707336.2
(22) Date of filing: 20.02.2020
(51) Int. Cl.: A61K 38/12, A23L 33/18, A61P 25/28

(54) **LYSOZYME HYDROLYSATE FOR PROACTIVE INHIBITORY CONTROL AND CONTROL OF IMPULSIVITY**
LYSOZYM-HYDROLYSAT ZUR PROAKTIVEN INHIBITIONSKONTROLLE UND IMPULSIVITÄTSSTEUERUNG
HYDROLYSAT DE LYSOZYME POUR UN CONTRÔLE INHIBITEUR PROACTIF ET UN CONTRÔLE DE L'IMPULSIVITÉ

(30) Priority: 22.02.2019 NL 2022627
(43) Date of publication of application: 29.12.2021
(73) Proprietor: Newtricious B.V., 5223 DE 's Hertogenbosch (NL)
(72) Inventor: PLAT, Jogchum, 6243 CP Geulle (NL); ADAM, Joseph Jacobus Maria Eugène, 6228 SP Maastricht (NL); MENSINK, Ronald Peter, 6101 KE Echt (NL); BIVERT, Marcel Alexander, 6226 GS Maastricht (NL); VAN DER MADE, Sanne Maria, 5258 BZ Berlicum (NL)
(74) Representative: Nederlandsch Octrooibureau
(86) International application number: PCT/NL2020/050102
(87) International publication number: WO 2020/171706

(56) References cited:
- WO-A1-2009/133055
- WO-A1-2017/144443
- ROBERT DEACON: "Assessing Burrowing, Nest Construction, and Hoarding in Mice", JOURNAL OF VISUALIZED EXPERIMENTS, no. 59, 1 January 2012 (2012-01-01), pages e2607 - 1, XP055244255, DOI: 10.3791/2607

## Description

### TECHNICAL FIELD

The invention relates to a non-therapeutic method of improving or maintaining cognitive functioning in a healthy subject, the method comprising administering to the subject a composition comprising subtilisin A hydrolysate of lysozyme, wherein said improving cognitive function is any one or more of improving inhibitory control and improving control of impulsivity. Furthermore, the invention relates to a subtilisin A hydrolysate of lysozyme for use in a method for the prophylaxis or treatment any of more of impaired inhibitory control and impaired control of impulsivity in a subject suffering from any one or more of a neurodegenerative disease, Alzheimer's disease, dementia, Parkinson's Disease, obsessive-compulsive disorder, compulsive neurosis, Tourette's syndrome, attention deficit hyperactivity disorder, a psychiatric disorder, Trichotillomania, schizophrenia, and an addiction to any one of a drug, a narcotics, nicotine, alcohol, gaming, and gambling.

### BACKGROUND

Nowadays society is an environment that is increasingly demanding almost continuous alertness and attention from human subjects. At school, work, university, while traveling through busy traffic, when at home and being exposed to several sources of (social) media; proper cognitive functioning is a prerequisite and a benefit to the subject who has to master all sources of signals, information, temptations and seduction. Subjects may constantly be at risk for poor reaction time when a task or circumstance is demanding faster reaction time. Social media, (video, internet) games, alcohol, cigarettes, real-life real-time social interactions and encounters, etc., may all be examples of circumstances requiring adequate control of impulsivity, suitable proactive inhibitory control and/or attention. Therefore, impulse control and sustained attention are both examples of core cognitive functions which play a critical role in daily functioning.

Healthy human subjects experience the natural gradual and steady decline of cognitive function over time when ageing, during adult life. Receiving, mastering and responding properly to all kinds and sources of information and signals thus demands an increasing effort as a consequence of ageing, since cognitive functions such as those related to inhibitory control, proactive inhibitory control, attention, sustained attention, control of impulsivity, alertness, sustained alertness, decline naturally over time. For example, elderly people may suffer from feeling lost or experience feelings of anxiousness when participating in (car) traffic, public transport, crowds, *etc.,* due to the decline of said cognitive functions. For example, during the years professionals such as teachers, guards, bus drivers, *etc.,* may experience uncomfortable effects of gradually declining cognitive functioning when intensively interacting with youngsters.

Apart from decline of cognitive functioning in healthy subjects as a fact of life, several health issues and disease states are correlated with a (risk for a) decline of cognitive functions such as inhibitory control, proactive inhibitory control, attention, sustained attention, control of impulsivity, alertness, sustained alertness. For example, declining cognitive function is seen with human patients suffering from one or more of hyperactivity, a neurodegenerative disease, Alzheimer's disease, dementia, Parkinson's Disease, obsessive-compulsive disorder, compulsive neurosis, Tourette's syndrome, attention deficit hyperactivity disorder, a psychiatric disorder, Trichotillomania, an addiction, such as addiction to a drug, a narcotics, nicotine, alcohol, gaming, gambling. Especially, declined or impaired proactive inhibitory control, in particular impaired control of impulsivity, are aspects hampering daily life of these patients. In addition, patients suffering from one or more of attention deficit hyperactivity disorder, a psychiatric disorder, schizophrenia, hyperactivity often experience the drawback of declined cognitive functions, for example related to deteriorated sustained attention, for example impaired general alertness.

Therefore, a solution still needs to be found that allows for improvement of cognitive functions in healthy subjects, such as cognitive function relating to inhibitory control, proactive inhibitory control, attention, sustained attention, control of impulsivity, alertness, sustained alertness. Also, a solution still needs to be found that allows for improvement of declined or declining cognitive functions in healthy subjects, such as cognitive function relating to inhibitory control, proactive inhibitory control, attention, sustained attention, control of impulsivity, alertness, sustained alertness, the decline relating to the natural phenomenon of deterioration of cognitive function as a consequence of ageing. In addition, a solution still needs to be found that allows for improving or maintaining cognitive functions in patients or halting or reversing decline of cognitive functions in patients, such as cognitive function relating to inhibitory control, proactive inhibitory control, attention, sustained attention, control of impulsivity, alertness, sustained alertness, the decline relating to a subject suffering from a health problem or disease accompanied by said declining cognitive functions.

International patent application WO2017/144443 relates to a composition for the prevention or treatment of neurodegenerative disease, in particular dementia such as Alzheimer's Disease. The composition comprises a lysozyme hydrolysate. The effect of the composition on ability to perform normal everyday tasks is assessed using a mouse burrowing test [1-3]. Burrowing by mice is an activity of daily living (ADL; care of the self, social and domestic environment), analogous to human ADLs. Disruption of the ability to perform ADL is an early sign of neurodegenerative diseases such as Alzheimer's Disease. International patent application WO2009133055 relates to a composition for use to prevent or to treat for example, alertness, vigilance, cognition, wherein the cognition is defined as the combined skills relating to such areas as problem solving, learning, memory and language. The composition disclosed in WO2009133055 can comprise hydrolyzed lysozyme. Effect of a composition on e.g. alertness and vigilance and cognition is assessed in WO2009133055 by applying the Mackworth Clock test [4] and the Critical Tracking Task [5, 6]. The Mackworth Clock test is designed to establish vigilance and sustained attention, wherein vigilance describes a psychological readiness to perceive and respond, which is a process that, unlike attention, need not necessarily be consciously experienced [4]. The Critical Tracking Task requires and tests focused attention and visual motor control [6].

### SUMMARY

It is a first goal of the present invention to provide a composition, food stuff, nutraceutical, feed product, *etc.,* for improving or maintaining (conserving) cognitive function. The cognitive function is preferably any one or more of inhibitory control, proactive inhibitory control, attention, sustained attention, control of impulsivity, alertness, more preferably any one or more of proactive inhibitory control, sustained attention, control of impulsivity. In particular, the cognitive function is inhibitory control, proactive inhibitory control, and/or control of impulsivity. More in particular, the cognitive function is all of inhibitory control, preferably proactive inhibitory control, attention, preferably sustained attention, control of impulsivity, alertness.

It is an objective of the current invention to provide a composition for improving or conserving cognitive function, the cognitive function being any one or more of inhibitory control, attention, control of impulsivity, alertness, proactive inhibitory control and sustained attention, in particular inhibitory control, proactive inhibitory control, and/or control of impulsivity. It is a further objective of the current invention to provide a composition for improving cognitive function in a healthy individual, wherein the individual is preferably a human subject. It is also an objective of the current invention to provide a composition for use in a method for the treatment of impaired cognitive functioning in a subject in need thereof, e.g. a human subject. Treating impaired cognitive function in a subject in need thereof is any one or more of reversing previously declined cognitive function, improving current status of cognitive function, ameliorating cognitive function, preserving or conserving cognitive function, *etc.,* wherein the cognitive function is any one or more of inhibitory control, proactive inhibitory control, attention, sustained attention, control of impulsivity, alertness, more preferably any one or more of proactive inhibitory control, sustained attention, control of impulsivity. In particular, the cognitive function is inhibitory control, proactive inhibitory control, and/or control of impulsivity. More in particular, the cognitive function is all of inhibitory control, preferably proactive inhibitory control, attention, preferably sustained attention, control of impulsivity, alertness.

At least one of the above objectives is achieved by providing a protein lysate of the invention. More in particular, at least one of the above objectives is achieved by providing a lysozyme lysate of the invention, the lysate being a subtilisin A hydrolysate of lysozyme. In particular, at least one of the above objectives is achieved by providing a composition comprising subtilisin A hydrolysate of lysozyme.

The invention is set out in the appended set of claims.

A first aspect of the invention relates to a non-therapeutic method of improving cognitive functioning in a subject, wherein said subject is a healthy human subject, the method comprising administering to the subject a composition comprising subtilisin A hydrolysate of lysozyme, wherein said improving cognitive function is any one or more of improving inhibitory control and improving control of impulsivity. Subtilisin A is a serine endopeptidase. The improvement of cognitive functioning is an increase of the quality of the cognitive functioning or an increase of the extent of the cognitive functioning to the benefit of the subject concerned and/or to the benefit of subjects interacting with the subject concerned, and/or the improvement of cognitive functioning is maintaining the cognitive functioning at the current level, thereby preventing deterioration or decline of cognitive functioning, such as decline of cognitive functioning as a consequence of the natural phenomenon of ageing of a subject.

The non-therapeutic method of improving cognitive functioning in a subject is any one or more of improving inhibitory control, improving control of impulsivity. In particular, improving inhibitory control is improving proactive inhibitory control. In the non-therapeutic method of the invention, wherein improving cognitive functioning is improving control of impulsivity and/or improving (proactive) inhibitory control. More preferred is the non-therapeutic method of the invention, wherein improving cognitive functioning is improving all of (proactive) inhibitory control, (sustained) attention, control of impulsivity and alertness.

Improvement of attention such as sustained attention and/or improvement of alertness, such as general alertness, under influence of consumption of the subtilisin A hydrolysate of lysozyme, as assessed with the application of the sustained attention test (SAT), i.e. a psychomotor vigilance test, as established for human subjects, is at least 0,5% for sustained attention and for of general alertness, such as at least 1%, at least 2%, at least 2,5%, at least 3%. For example, the attention such as sustained attention and/or the alertness such as general alertness improve with 0.5%-10%, or 1%-3%, under influence of daily consumption of subtilisin A hydrolysate of lysozyme, such as the daily consumption of a food product comprising subtilisin A hydrolysate of lysozyme. In particular, said improvements are achieved when human subjects such as healthy human subjects consume the subtilisin A hydrolysate of lysozyme, according to the invention.

Improvement of cognitive control such as proactive cognitive control (or proactive inhibitory control) and/or improvement of impulse control (control of impulsivity), under influence of consumption of the subtilisin A hydrolysate of lysozyme, as assessed with the application of the anticue task ["Switch hands! Mapping temporal dynamics of proactive manual control with anticues", J.J. Adam, S. Jennings, T.J.H. Bovend'Eerdt, P.P.M. Hurks, P.W.M. Van Gerven, Acta Psychologica 161 (2015), pp.137-144], as established for human subjects, is at least 0,5% for cognitive control such as proactive cognitive control and for impulse control, such as at least 1%, at least 2%, at least 2,5%, at least 3%. For example, the proactive cognitive control and/or the impulse control improve with 0.5%-10%, or 2%-3%, under influence of daily consumption of subtilisin A hydrolysate of lysozyme, such as the daily consumption of a food product comprising subtilisin A hydrolysate of lysozyme. Typically, the proactive cognitive control is proactive inhibitory control.

In embodiments, in the non-therapeutic method of improving cognitive functioning in a healthy human subject, the subject is a hyperactive subject. The healthy subject may diagnosed with metabolic syndrome.

In the non-therapeutic method of improving cognitive functioning in a subject, the subject is a human male, more preferably a human male at least 60 years of age, preferably at least 65 years of age, more preferably at least 67 years of age, such as 60-105 years of age or 67-75 years of age. One embodiment is the non-therapeutic method of improving cognitive functioning in a subject, wherein the subject is a healthy human subject at least 60 years of age, preferably at least 65 years of age, more preferably at least 67 years of age, such as 60-105 years of age or 67-75 years of age.

A further aspect of the invention relates to a subtilisin A hydrolysate of lysozyme for use in a method for the prophylaxis or treatment any one or more of impaired inhibitory control and impaired control of impulsivity in a subject suffering from any one or more of a neurodegenerative disease, Alzheimer's disease, dementia, Parkinson's Disease, obsessive-compulsive disorder, compulsive neurosis, Tourette's syndrome, attention deficit hyperactivity disorder, a psychiatric disorder, Trichotillomania, schizophrenia, and an addiction to any one of a drug, a narcotics, nicotine, alcohol, gaming, and gambling. Preferred is subtilisin A hydrolysate of lysozyme for use in the method, wherein the impaired cognitive functioning is impaired proactive inhibitory control. Improvement of cognitive control such as proactive cognitive control (or proactive inhibitory control) and/or improvement of impulse control (control of impulsivity), under influence of consumption of the subtilisin A hydrolysate of lysozyme, as assessed with the application of the anticue task ["Switch hands! Mapping temporal dynamics of proactive manual control with anticues", J.J. Adam, S. Jennings, T.J.H. Bovend'Eerdt, P.P.M. Hurks, P.W.M. Van Gerven, Acta Psychologica 161 (2015), pp.137-144], as established for human subjects, is at least 0,5% for cognitive control such as proactive cognitive control and for impulse control, such as at least 1%, at least 2%, at least 2,5%, at least 3%. For example, the proactive cognitive control and/or the impulse control improve with 0.5%-10%, or 2%-3%, under influence of daily consumption of subtilisin A hydrolysate of lysozyme, such as the daily consumption of a food product comprising subtilisin A hydrolysate of lysozyme. Typically, the proactive cognitive control is proactive inhibitory control.

The subtilisin A hydrolysate of lysozyme for use in the method according to claim 1, is wherein the subject in need thereof suffers from any one or more of a neurodegenerative disease, Alzheimer's disease, dementia, Parkinson's Disease, obsessive-compulsive disorder, compulsive neurosis, Tourette's syndrome, attention deficit hyperactivity disorder, a psychiatric disorder, Trichotillomania, an addiction to a drug, a narcotics, nicotine, alcohol, gaming, gambling. The subject in need thereof is preferably a human subject. The impaired cognitive functioning to be treated is impaired inhibitory control, impaired proactive inhibitory control and/or impaired control of impulsivity, and is more in particular the combination of impaired inhibitory control, impaired proactive inhibitory control, impaired control of impulsivity, deteriorated attention, deteriorated sustained attention and impaired alertness.

In other examples not claimed is the subtilisin A hydrolysate of lysozyme for use in the method for the treatment of impaired cognitive functioning in a subject in need thereof, wherein the impaired cognitive functioning is any one or more of deteriorated attention, deteriorated sustained attention and impaired alertness. Improvement of attention such as sustained attention and/or improvement of alertness, such as general alertness, under influence of consumption of the subtilisin A hydrolysate of lysozyme, as assessed with the application of the sustained attention test (SAT), i.e. a psychomotor vigilance test, as established for human subjects, is at least 0,5% for sustained attention and for of general alertness, such as at least 1%, at least 2%, at least 2,5%, at least 3%. For example, the attention such as sustained attention and/or the alertness such as general alertness improve with 0.5%-10%, or 1%-3%, under influence of daily consumption of subtilisin A hydrolysate of lysozyme, such as the daily consumption of a food product comprising subtilisin A hydrolysate of lysozyme. In particular, said improvements are achieved when human subjects such as healthy human subjects consume the subtilisin A hydrolysate of lysozyme, according to the invention.

In particular, the subtilisin A hydrolysate of lysozyme for use in the method for the treatment of impaired cognitive functioning in a subject in need thereof is for use in the method according to claim 1, wherein the subject in need thereof suffers from any one or more of attention deficit hyperactivity disorder, a psychiatric disorder, schizophrenia. The subject in need thereof is preferably a human subject. The impaired cognitive functioning to be treated is more in particular the combination of impaired inhibitory control, impaired proactive inhibitory control, impaired control of impulsivity, deteriorated attention, deteriorated sustained attention and impaired alertness.

One embodiment is the subtilisin A hydrolysate of lysozyme for use in the method for the treatment of impaired cognitive functioning in a subject in need thereof, wherein the subject is male, preferably a human male, more preferably a human male at least 60 years of age, preferably at least 65 years of age, more preferably at least 67 years of age, such as 60-105 years of age or 67-75 years of age.

The inventors surprisingly found that daily consumption for at least four weeks of the subtilisin A hydrolysate of lysozyme improved cognitive function, in particular inhibitory control and/or control of impulsivity, more in particular the combination of inhibitory control, proactive inhibitory control, control of impulsivity, attention, sustained attention and alertness. Reference is made to the examples in the Examples section, here below.

According to embodiments of the invention, in the non-therapeutic method of improving cognitive functioning in a subject, at least 30% by weight of the subtilisin A hydrolysate of lysozyme are lysozyme peptides with a molecular weight of less than 0,5 kDa. Furthermore, for the subtilisin A hydrolysate of lysozyme for use in the method for the treatment of impaired cognitive functioning in a subject in need thereof, at least 30% by weight of the subtilisin A hydrolysate of lysozyme are lysozyme peptides with a molecular weight of less than 0,5 kDa.

Preferred is the non-therapeutic method of improving cognitive functioning in a subject according to the attached claims, the method comprising administering to the subject a composition comprising subtilisin A hydrolysate of lysozyme, wherein the degree of hydrolysis of the lysozyme is at least 12%, preferably 15% - 50%, more preferably 18% - 40%, most preferably 19% - 30%, such as 21%. Also preferred is the subtilisin A hydrolysate of lysozyme for use in the method for the treatment of impaired cognitive functioning in a subject in need thereof according to the attached claims, wherein the degree of hydrolysis of the lysozyme is at least 12%, preferably 15% - 50%, more preferably 18% - 40%, most preferably 19% - 30%, such as 21%.

For a preferred non-therapeutic method of improving cognitive functioning in a subject, the daily dose of the subtilisin A hydrolysate of lysozyme is 0,5 gram - 20 gram per day, preferably 1 gram - 10 gram per day, more preferably 2 gram - 8 gram per day, such as 5 gram per day. The daily dose is preferably administered to the subject as a single daily dose. Preferred is the subtilisin A hydrolysate of lysozyme for use in the method for the treatment of impaired cognitive functioning in a subject in need thereof, wherein the daily dose of the subtilisin A hydrolysate of lysozyme is 0,5 gram - 20 gram per day, preferably 1 gram - 10 gram per day, more preferably 2 gram - 8 gram per day, such as 5 gram per day. The daily dose is preferably administered to the subject as a single daily dose. Preferred is a daily dose, such as a single daily dose, of less than 10 gram, such as 1 - 9,5 gram/daily. Preferably, a human subject such as a healthy human subject is administered a daily dose of the subtilisin A hydrolysate of lysozyme of between 0,5 gram and less than 10 gram, although higher doses are also applicable, such as up to 20 gram. Typically, the daily dose is administered for at least 4 weeks to the subject in need thereof, e.g. to a (healthy) human subject, preferably at least for 8 weeks, 16 weeks, 40 weeks, 1 year, 2 years, 5 years, for the remaining life time of the subject, according to the invention. Beneficial effects on cognitive function (e.g. improved control of impulsivity, improved inhibitory control) are achieved after at least 4 weeks of daily consumption of 0,5 - 20 gram of the subtilisin A hydrolysate of lysozyme, preferably 0,5 - less than 10 gram, according to the invention. It is part of the invention that the subtilisin A hydrolysate of lysozyme is provided once daily or for example twice daily in for example two equal doses, accumulating to 0,5 - 20 gram/daily, or 1 - less than 10 gram/daily.

An aspect of the current invention relates to the use of a nutraceutical, a food product, a functional food, a food stuff, a food ingredient, a food supplement, comprising a subtilisin A hydrolysate of lysozyme, in the non-therapeutic method of improving cognitive functioning in a subject according to the invention. The subtilisin A hydrolysate of lysozyme is the hydrolysate of any of the aforementioned aspects and embodiments of the invention.

An aspect of the current invention relates to a nutraceutical, a food product, a functional food, a food stuff, a food ingredient, a food supplement and/or a feed product, comprising a subtilisin A hydrolysate of lysozyme, for use in the method for the treatment of impaired cognitive functioning in a subject in need thereof according to the invention. The subtilisin A hydrolysate of lysozyme is the hydrolysate of any of the aforementioned aspects and embodiments of the invention.

### BRIEF DESCRIPTION OF THE DRAWING

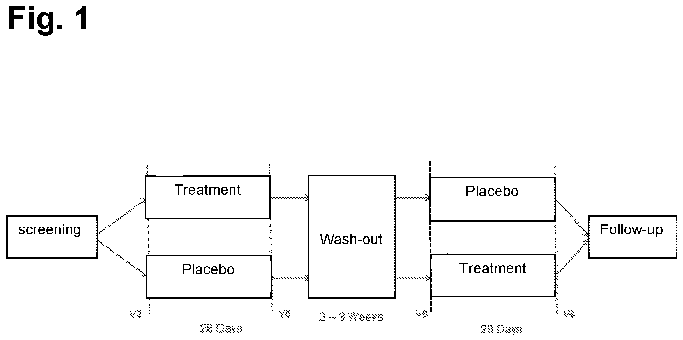

Fig. 1 displays the flow chart of the study design, the study being a randomized, double-blind, placebo controlled, cross-over clinical trial.

### DEFINITIONS

The term "inhibitory control", or "response inhibition", as used herein has its regular scientific meaning, and here refers to the cognitive process, more specifically the executive function, that permits individuals to inhibit their impulses and natural, habitual, or dominant behavioral responses to stimuli in order to select a more appropriate behavior that is consistent with completing their goals. Self-control is an important aspect of inhibitory control. "Improving inhibitory control" thus relates to an increased inhibition of a subject's impulses. "Impaired inhibitory control" thus relates to decreased possibility for a subject to inhibit impulses and to less possibility to inhibit natural, habitual, or dominant behavioral responses to stimuli in order to select a more appropriate behavior that is consistent with completing their goals.

The term "attention" as used herein has its regular scientific meaning, and here refers to the behavioral and cognitive process of selectively concentrating on a discrete aspect of information, whether deemed subjective or objective, while ignoring other perceivable information. It is a state of arousal. It is the taking possession by the mind in clear and vivid form of one out of what seem several simultaneous objects or trains of thought. Focalization, the concentration of consciousness, is of its essence. "Improving attention" thus relates to increasing the concentration of consciousness and rising selective concentration.

The term "impulsivity", or "impulsiveness", as used herein has its regular scientific meaning, and here refers to the tendency to act on a whim, displaying behavior characterized by little or no forethought, reflection, or consideration of the consequences. Impulsive actions are typically poorly conceived, prematurely expressed, unduly risky, or inappropriate to the situation that often result in undesirable consequences, which imperil long-term goals and strategies for success. "Improving control impulsivity" thus amongst others refers to dampening of the tendency to act on a whim. "Impaired control of impulsivity" thus refers to the condition that a subject has an increased tendency to act on a whim, increasingly displaying behavior characterized by little or no forethought, reflection, or consideration of the consequences.

The term "alertness" as used herein has its regular scientific meaning, and here refers to the state of active attention by high sensory awareness such as being watchful and prompt to meet danger or emergency, or being quick to perceive and act. Alertness is related to psychology as well as to physiology. "Improving alertness" thus relates to strengthening the state of active attention, increasing sensory awareness. "Impaired alertness" thus refers to a shorter state of active attention or no state of active attention at all.

The term "proactive inhibitory control" as used herein has its regular scientific meaning, and here refers to how a subject prepares to stop an upcoming response tendency, i.e. proactive stopping. The stopping refers to the behavioral outcome when a subject halts an incipient response tendency. "Improving proactive inhibitory control" thus relates to a more efficient and/or faster preparation to stop an upcoming response tendency. "Impaired proactive inhibitory control" thus relates to a decreased possibility or tendency, or even no possibility or tendency at all, for a person to prepare to stop an upcoming response tendency, resulting in delayed proactive stopping or absence of proactive stopping.

The term "sustained attention", or "attention span", as used herein has its regular scientific meaning, and here refers to the amount of concentrated time a person can spend on a task without becoming distracted. The element of distractibility occurs when the individual is uncontrollably drawn to some other activity or sensation. The ability to focus and sustain attention on a task is crucial for the achievement of one's goals. "Improving sustained attention" thus relates to increasing the amount of concentrated time a person can spend on a task without becoming distracted and/or to increasing the ease with which a person can spend time on a task without becoming distracted. "Deteriorated sustained attention" thus refers to the circumstances that a person can spend less concentrated time on a task without being distracted, or even no concentrated time at all.

The term "food" has its regular scientific meaning throughout the text, and here refers to liquid, semi-liquid or solid food products suitable for human and/or animal consumption.

The term "functional food" has its regular scientific meaning throughout the text, and here refers to a food product which comprises one or more active ingredients, especially with subtilisin A hydrolysate of lysozyme according to the invention as active ingredient.

The term "food supplement" has its regular scientific meaning throughout the text, and here refers to supplements suitable for human and/or animal consumption which comprise a suitable amount of one or more subtilisin A hydrolysate of lysozyme according to the invention as functional ingredient. Supplements may be in the form of pills, sachets, powders and the like.

The term "subject" has its regular scientific meaning throughout the text, and here refers to any member of the class mammals, including without limitation humans, non-human primates, farm animals, domestic animals and laboratory animals.
The term "lysozyme hydrolysate' is used herein as a general term to refer to hydrolysates, for example prepared in vitro, of lysozyme.

"Metabolic Syndrome" refers to multiple interrelated clinical disorders, including obesity, insulin resistance and hyperinsulinemia, glucose intolerance, hypertension and dyslipidemia (hypertriglyceridemia and low HDL cholesterol levels) as described e.g. in Moller and Kaufman (Annual Rev. of Medicine 2005, vol 56:45-62). In the context of the current invention, preferably metabolic syndrome is diagnosed by an impaired glucose regulation/insulin resistance and ≥ 2 other criteria from 1) Impaired glucose regulation/insulin resistance 2) abdominal obesity 3) hypertriglyceridemia 4) low levels of HDL cholesterol 5) microalbuminuria, wherein the clinical features are further defined according to the WHO definitions as described in Moller and Kaufman.

"Degree of hydrolysis" is the proportion of cleaved peptide bonds in a protein hydrolysate.

### DETAILED DESCRIPTION

While the invention has been described in terms of several embodiments, it is contemplated that alternatives, modifications, permutations and equivalents thereof will become apparent to one having ordinary skill in the art upon reading the specification and upon study of the drawings. The invention is not limited in any way to the illustrated embodiments. Changes can be made without departing from the scope which is defined by the appended claims.

The present invention will be described with respect to particular embodiments but the invention is not limited thereto but only by the claims.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

The embodiments of the invention described herein can operate in combination and cooperation, unless specified otherwise.

Furthermore, the various embodiments, although referred to as "preferred" or "e.g." or "for example" or "in particular" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a composition comprising A and B" should not be limited to the composition consisting only of components A and B, rather with respect to the present invention, the only enumerated components of the composition are A and B, and further the claim should be interpreted as including equivalents of those components.

In addition, reference to an element by the indefinite article "a" or "an" does not exclude the possibility that more than one of the element are present, unless the context clearly requires that there is one and only one of the elements. The indefinite article "a" or "an" thus usually means "at least one". The inventors surprisingly found in a randomized double-blind, placebo controlled, cross-over study that administering a composition comprising a protein lysate to human subjects resulted in improved cognitive functioning. In particular, the inventors found in said clinical trial that cognitive functioning relating to inhibitory control and/or to attention, such as proactive inhibitory control and sustained attention, is/are improved in human subjects to whom an effective amount of subtilisin A hydrolysate of lysozyme is administered.

A first aspect of the invention relates to a non-therapeutic method of improving cognitive functioning in a subject, wherein said subject is a healthy human subject, the method comprising administering to the subject a composition comprising subtilisin A hydrolysate of lysozyme, wherein said improving cognitive function is any one or more of improving inhibitory control and improving control of impulsivity. In particular an effective amount of subtilisin A hydrolysate of lysozyme is administered to the subject. For example, the subject is in need of direct improvement of cognitive functioning. The inventors surprisingly found that daily intake of a composition comprising subtilisin A hydrolysate of lysozyme resulted in improved cognitive functioning. Improvements were already evident after a time course of daily intake of four weeks. Subjects in a cross-over clinical trial showed improvement in cognitive functioning, i.e. inhibitory control, attention, control of impulsivity, alertness, after four weeks of intake of 5 grams of subtilisin A hydrolysate of lysozyme. After a wash-out period of 2-8 weeks, most often 4 weeks, switching to daily intake of placebo resulted in decline of the cognitive functioning to a level determined at the start of the first 4-weeks period of intake (See Fig. 1 for the scheme of the set-up of the clinical trial performed). In turn, subjects starting with daily intake of placebo for 4 weeks did not show an improvement in cognitive functioning, whereas switching to the daily intake of a composition comprising subtilisin A hydrolysate of lysozyme, e.g. 200 ml of an aqueous solution comprising 5 grams of hydrolysate, resulted in improvement in cognitive functioning after 4 weeks, compared to cognitive functioning measures determined at the start of the 4 weeks and compared to subjects to whom placebo was administered during the same course of time.

According to the invention the non-therapeutic method of improving cognitive functioning in a subject, wherein improving cognitive functioning is any one or more of improving inhibitory control, improving control of impulsivity, and wherein the subject is a healthy human subject. Even more preferred is the non-therapeutic method of improving cognitive functioning in a subject, wherein improving cognitive functioning is all of improving inhibitory control, improving attention, improving control of impulsivity and improving alertness.

In particular, improving inhibitory control is improving proactive inhibitory control, and/or improving attention is improving sustained attention.

In the non-therapeutic method of improving cognitive functioning in a subject according to the invention, the subject is a healthy human subject. The subject is for example a healthy human subject who is a hyperactive subject.

Improvement of attention such as sustained attention and/or improvement of alertness, such as general alertness, under influence of consumption of the subtilisin A hydrolysate of lysozyme, as assessed with the application of the sustained attention test (SAT), i.e. a psychomotor vigilance test, as established for human subjects, is at least 0,5% for sustained attention and for of general alertness, such as at least 1%, at least 2%, at least 2,5%, at least 3%. For example, the attention such as sustained attention and/or the alertness such as general alertness improve with 0.5%-10%, or 1%-3%, under influence of daily consumption of subtilisin A hydrolysate of lysozyme, such as the daily consumption of a food product comprising subtilisin A hydrolysate of lysozyme.

Improvement of cognitive control such as proactive cognitive control and/or improvement of impulse control, under influence of consumption of the subtilisin A hydrolysate of lysozyme, as assessed with the application of the anticue task, as established for human subjects, is at least 0,5% for cognitive control such as proactive cognitive control and for of impulse control, such as at least 1%, at least 2%, at least 2,5%, at least 3%. For example, the proactive cognitive control and/or the impulse control improve with 0.5%-10%, or 2%-3%, under influence of daily consumption of subtilisin A hydrolysate of lysozyme, such as the daily consumption of a food product comprising subtilisin A hydrolysate of lysozyme.

Daily intake of the composition comprising subtilisin A hydrolysate of lysozyme results in improved cognitive functioning in the domains of cognition related to self-control, control of impulsivity, maintaining attention, staying alert. In particular, daily intake of the composition comprising subtilisin A hydrolysate of lysozyme results in improved cognitive functioning in the domains of cognition related to self-control, control of impulsivity. Therefore, for a subject, preferably a healthy subject, e.g. a healthy human subject, the benefits of the daily intake of the composition comprising subtilisin A hydrolysate of lysozyme are manifold and numerous. In particular, when the subtilisin A hydrolysate of lysozyme is consumed (daily) by the human subject for at least 4 weeks, preferably at least 0,5 gram/daily and up to 20 gram daily, preferably less than 10 gram/daily such as about 5 gram/daily. For example, improved and increased alertness is beneficial in a plethora of daily circumstances like during maneuvering through crowded traffic, during taking classes at school, workshops at work, business meetings, lectures at university or at a conference, during an overnight truck drive or intercontinental plane flight, or for guards, or when working as ground-controller at an airport or in a harbor, while executing complex surgery, during an extended trial in court, *etc.*, *etc.* Improved control of impulsivity may contribute to overall improved quality of life, both for the subject hem/herself and subjects interacting with him/her. For example, improved control of impulsivity may reduce aggression, for example during daily traffic, in crowds, *etc.* Similar to the benefits for the subject related to improved alertness, improved attention, such as prolonged and/or uninterrupted attention and/or more focused, intense attention, also provides numerous and similar benefits to the subject.

Further benefits of the improved attention such as sustained attention and/or alertness, such as general alertness, and/or cognitive control such as proactive cognitive control and/or impulse control, under influence of consumption, preferably daily consumption, preferably for at least 4 weeks, of the subtilisin A hydrolysate of lysozyme, are evident for, for example, students such as school children or university students, while for example studying and preparing for exams and while taking lectures and courses and sitting exams, thus in general when an intellectual task has to be executed. Typically the student is 17-26 years of age such as 18-24 years of age. Improved attention such as sustained attention and/or alertness, such as general alertness, and/or cognitive control such as proactive cognitive control and/or impulse control helps the student in learning and/or in absorbing new knowledge while for example taking classes, and furthermore helps the student during reproducing gained knowledge for example while taking an exam or when subjected to (oral) examination.

Further benefits of the improved attention such as sustained attention and/or alertness, such as general alertness, and/or cognitive control such as proactive cognitive control and/or impulse control, under influence of consumption, preferably daily consumption, of the subtilisin A hydrolysate of lysozyme, are evident for, for example, sportsmen/sportswomen, when sporting or training for competition or when in competition at any level and in particularly when sporting or training for competition or when in competition at the top level and/or when acting on tournaments, such as the Olympic games, world championships, etc. Improved attention such as sustained attention and/or alertness, such as general alertness, and/or cognitive control such as proactive cognitive control and/or impulse control supports the sportsmen or sportswomen while exercising for example as a daily routine and when taking several hours per day requiring dedication and endurance, and helps the sportsmen or sportswomen while focusing on e.g. a win during competition and when participating in a match.

Further benefits of the improved attention such as sustained attention and/or alertness, such as general alertness, and/or cognitive control such as proactive cognitive control and/or impulse control, under influence of consumption, preferably daily consumption, of the subtilisin A hydrolysate of lysozyme, are evident for, for example, children, youngsters, young adults and adults suffering from ADHD, in particular young adults suffering from ADHD, for example young adults 14-22 years of age, preferably 15-20 years of age or 16-20 years of age such as 16-18 years of age. Improved attention such as sustained attention and/or alertness, such as general alertness, and/or cognitive control such as proactive cognitive control and/or impulse control, is beneficial for a subject suffering from ADHD, such as a young adult suffering from ADHD, while for example taking classes at school, while focusing on home work, study, hobby, social interactions, a game, taking dinner, etc.

An embodiment is the non-therapeutic method of improving cognitive functioning in a subject, wherein the subject is a human subject selected from a student such as a school child or a university student, a sportsman or sportswoman, such as a top sportsman or top sportswoman, and from a child, a youngster, a young adult.

The inventors found to their surprise that daily consumption of the composition comprising subtilisin A hydrolysate of lysozyme, e.g. for at least 4 weeks, e.g. at a daily dose of less than 10 gram/daily, such as 1 - 9 gram/daily, had a beneficial effect on cognitive functioning to the extent of reversing about 1 to 2 years of natural decline of cognitive functioning due to normal ageing of a human subject. That is to say, the size and extent of improvement in any one or more of control of impulsivity, alertness, proactive inhibitory control and sustained attention after consumption of the composition comprising subtilisin A hydrolysate of lysozyme was similar to the cognitive functioning of a subject as if he or she was one to two years younger. Decline of cognitive functioning over the course of adult life is normal and a natural phenomenon. By daily consumption of the composition comprising subtilisin A hydrolysate of lysozyme, this natural detoriation of cognitive functioning is delayed by one to two years. One embodiment is the non-therapeutic method of improving cognitive functioning in a subject, wherein the subject is a human adult subject at least 18 years of age. Thus, improved control of impulsivity and/or improved inhibitory control equaling a reversal of decline in control of impulsivity and/or reversal of decline in control of impulsivity due to regular aging of a human subject to an extent of 1-2 years is achieved by daily consumption of less than 10 gram subtilisin A hydrolysate of lysozyme for at least 4 weeks, according to the invention.

Furthermore, the disclosure concerns a non-therapeutic method of improving cognitive functioning in a healthy human subject diagnosed with metabolic syndrome, wherein a composition comprising subtilisin A hydrolysate of lysozyme is administered to the subject. In particular an effective amount of subtilisin A hydrolysate of lysozyme is administered to the subject.

Preferably the subject to whom the composition comprising subtilisin A hydrolysate of lysozyme is administered is in need of maintaining or improving cognitive function or is in need of prevention of decline of cognitive functioning. As said, daily intake by a human subject of the composition comprising subtilisin A hydrolysate of lysozyme improves cognitive functioning and reverses natural decline of cognitive functioning occurring over the course of 1-2 years. In an embodiment, the human subject is a healthy subject, wherein the subject is 50 years of age or older, such as 60 years of age or older, 65-100 years of age, over 75 years of age. Preferably, the subject is in need of amelioration or reversal of previously declined cognitive function, i.e. improvement of previously declined or deteriorated cognitive function such as proactive inhibitory control, control of impulsivity. Typically, the subject is a human subject 65 years of age or older. Also preferred is the non-therapeutic method of improving cognitive functioning in a subject, wherein the subject is a human child, 18 years of age or younger, or wherein the subject is a human subject 18-65 years of age. For example, the human subject is a student such as a university student, a youngster, a young adult, a young adult or adult suffering from ADHD, a sportsman or sportswoman, in particular a top sportsman or a top sportswoman.

Without wishing to be bound by theory, the improved cognitive functioning in subjects to whom the composition comprising subtilisin A hydrolysate of lysozyme is administered is related to improved peripheral vascular function induced by the intake of the composition comprising subtilisin A hydrolysate of lysozyme. The improved cognitive functioning observed in human subjects is amongst other improvements improved impulse control and improved sustained attention. The inventors determined these positive effects on cognitive functioning of the intake of the composition comprising subtilisin A hydrolysate of lysozyme in a series of cognitive function tests, as detailed in the Example section below: the sustained attention test (SAT) known in the art, a vigilance task designed to assess the ability of individuals to sustain attention, and the anticue task known in the art, a reaction time task, which assesses proactive cognitive control, especially relating to impulse control. Surprisingly, the observed improvement of cognitive functioning in the human subjects subjected to the clinical trial and daily consuming the composition comprising subtilisin A hydrolysate of lysozyme, again declined after daily intake was halted (subjects switched to daily intake of placebo after a 2-8 weeks wash-out period). Thus, again without wishing to be bound by theory, sustained improvement of the cognitive functioning such as control of impulsivity, proactive inhibitory control, alertness, sustained attention, occurs and is maintained when the composition comprising subtilisin A hydrolysate of lysozyme is consumed daily and chronically. Prolonged daily intake, e.g. for at least 4 weeks, for example by a human subject, of the composition comprising subtilisin A hydrolysate of lysozyme, preferably less than 10 gram/daily such as 0,5 - 9,5 gram daily, then maintains improved peripheral vascular function relating to proper blood circulation through the brain, ultimately resulting in the observed beneficial effects of the composition on cognitive functioning.

An embodiment is the non-therapeutic method of improving cognitive functioning in a healthy human subject, wherein improving cognitive functioning is improvement of proactive inhibitory control. An embodiment is the non-therapeutic method, wherein the proactive inhibitory control is control of impulsivity.

Particularly, in the non-therapeutic method of improving cognitive functioning in a subject, the subject is male, preferably a human male, more preferably a human male at least 60 years of age, preferably at least 65 years of age, more preferably at least 67 years of age, such as 60-105 years of age or 67-75 years of age. Particularly elderly men 67 years of age or older benefit from the cognitive function improving effects of daily intake of the composition comprising subtilisin A hydrolysate of lysozyme. Comparing men and women divided over six groups based on gender and age (55 years of age or younger, 56-66 years of age, 67 years of age or older), the men 67 years of age or older benefit to a larger extent from the daily intake of the composition comprising subtilisin A hydrolysate of lysozyme. The beneficial effect was about twice as large for these elder men.

A further aspect of the disclosure relates to subtilisin A hydrolysate of lysozyme for use in a method for the treatment of impaired cognitive functioning in a subject in need thereof. The subject can be a subject diagnosed with metabolic syndrome, i.e. a human subject. According to an example not claimed is the use of subtilisin A hydrolysate of lysozyme or a composition comprising a subtilisin A hydrolysate of lysozyme for the manufacture of a (nutritional) composition for treating impaired cognitive functioning in a subject, the subject preferably being a human subject. In particular an effective amount of subtilisin A hydrolysate of lysozyme is administered to the subject. For example, the subject is in need of direct improvement of cognitive functioning. For example, the subject is in need of direct amelioration or reversal of previously declined cognitive functioning. Furthermore, in an embodiment of the method, preferred is the subtilisin A hydrolysate of lysozyme for use in a method for the prevention, prophylaxis, amelioration, or slowing down further decline of impaired cognitive functioning in a subject in need thereof. Also in an embodiment of the method, preferred is the subtilisin A hydrolysate of lysozyme for use in a method for the prevention of decline of cognitive functioning, prophylaxis of cognitive functioning, amelioration of declined cognitive functioning, or slowing down decline of cognitive functioning in a subject in need thereof. In an embodiment of the method, the method also concerns a method for improving or reversing impaired cognitive functioning in a subject suffering from said impaired cognitive functioning, wherein a composition comprising subtilisin A hydrolysate of lysozyme is administered to the subject. In particular an effective amount of subtilisin A hydrolysate of lysozyme is administered to the subject. Preferably the subject suffering from said impaired cognitive functioning is in need of (direct) improvement of cognitive functioning or is in need of (direct) halting of (further) decline of cognitive function.

Preferred is subtilisin A hydrolysate of lysozyme for use in the method for the treatment of impaired cognitive functioning in a subject in need thereof, wherein the impaired cognitive functioning is any one or more of impaired inhibitory control, impaired proactive inhibitory control and impaired control of impulsivity.

Also preferred is subtilisin A hydrolysate of lysozyme for use in the method for the treatment of impaired cognitive functioning in a subject in need thereof, wherein the cognitive functioning is any one or more of inhibitory control, proactive inhibitory control and control of impulsivity. Preferably, the subject in need thereof is a human subject. Preferably, the subject in need thereof is administered daily a dose of the subtilisin A hydrolysate of lysozyme of 0,5 - 20 gram, preferably less than 10 gram/daily, and preferably, the subject in need thereof is administered daily a dose of the subtilisin A hydrolysate of lysozyme, as a single dose or as multiple doses/day, such as 2 half-doses/day, for at least 4 weeks, such as at least 6 months, or 4 weeks - 10 years, or for the rest of the life time of the subject, such as a human subject.

Improvement of attention such as sustained attention and/or improvement of alertness, such as general alertness, under influence of consumption of the subtilisin A hydrolysate of lysozyme, as assessed with the application of the sustained attention test (SAT), i.e. a psychomotor vigilance test, as established for human subjects, is at least 0,5% for sustained attention and for of general alertness, such as at least 1%, at least 2%, at least 2,5%, at least 3%. For example, the attention such as sustained attention and/or the alertness such as general alertness improve with 0.5%-10%, or 1%-3%, under influence of daily consumption of subtilisin A hydrolysate of lysozyme, such as the daily consumption of a food product comprising subtilisin A hydrolysate of lysozyme.

Improvement of cognitive control such as proactive cognitive control and/or improvement of impulse control, under influence of consumption of the subtilisin A hydrolysate of lysozyme, as assessed with the application of the anticue task, as established for human subjects, is at least 0,5% for cognitive control such as proactive cognitive control and for of impulse control, such as at least 1%, at least 2%, at least 2,5%, at least 3%. For example, the proactive cognitive control and/or the impulse control improve with 0.5%-10%, or 2%-3%, under influence of daily consumption of subtilisin A hydrolysate of lysozyme, such as the daily consumption of a food product comprising subtilisin A hydrolysate of lysozyme.

In particular, a composition comprising subtilisin A hydrolysate of lysozyme or the subtilisin A hydrolysate of lysozyme is for use in the method for the treatment of impaired cognitive functioning in a subject in need thereof, wherein the subject in need thereof suffers from any one or more of a neurodegenerative disease, Alzheimer's disease, dementia, Parkinson's Disease, obsessive-compulsive disorder, compulsive neurosis, Tourette's syndrome, attention deficit hyperactivity disorder, a psychiatric disorder, Trichotillomania, an addiction to a drug, a narcotics, nicotine, alcohol, gaming, gambling. In an example not claimed, the subtilisin A hydrolysate of lysozyme is for use in the method for the treatment of impaired cognitive functioning in a subject in need thereof, wherein the subject in need thereof suffers from any disease accompanied by declined cognitive functioning, deteriorated cognitive functioning, impaired cognitive functioning. For example, the subject such as a human subject suffers from declined cognitive functioning due to (previous) trauma, infection, inflammation, ischemia, stroke, cardiovascular disease, *etc.* The cognitive functioning is in particular inhibitory control and/or control of impulsivity, and preferably the cognitive functioning is the combination of inhibitory control, control of impulsivity, alertness, attention.

In an example not claimed is the subtilisin A hydrolysate of lysozyme or the composition comprising subtilisin A hydrolysate of lysozyme for use in the method for the treatment of impaired cognitive functioning in a subject in need thereof, wherein the impaired cognitive functioning is any one or more of deteriorated or impaired attention, deteriorated or impaired sustained attention and impaired or deteriorated alertness.

In particular, the subtilisin A hydrolysate of lysozyme for use in the method for the treatment of impaired cognitive functioning in a subject in need thereof is for use in the method, wherein the subject in need thereof suffers from any one or more of attention deficit hyperactivity disorder, a psychiatric disorder, schizophrenia. In an example not claimed, the subtilisin A hydrolysate of lysozyme for use in the method for the treatment of impaired cognitive functioning in a subject in need thereof is for use in the method, wherein the subject in need thereof suffers from any disease or disorder accompanied by impaired attention, deteriorated or impaired sustained attention and impaired or deteriorated alertness other than these aforementioned health issues and diseases.

Preferably, the subject is a human subject, such as a human subject 50 years of age or older, such as 60 years of age or older, 65-100 years of age, over 75 years of age. In case of the non-therapeutic method, the subject is a healthy subject. Preferably, the subject is in need of amelioration or reversal of previously declined cognitive function, i.e. improvement of previously declined or deteriorated cognitive function such as sustained attention, proactive inhibitory control, alertness, control of impulsivity. In particular, the subject is in need of amelioration or reversal of previously declined cognitive function, i.e. improvement of previously declined or deteriorated cognitive function such as proactive inhibitory control, control of impulsivity, or is in need of amelioration or reversal of previously declined cognitive function, i.e. improvement of previously declined or deteriorated cognitive function such as the combination of sustained attention, proactive inhibitory control, alertness and control of impulsivity. Typically, the subject is a human subject 65 years of age or older. In case of the non-therapeutic method, the human subject is a healthy human subject. Also preferred is the subtilisin A hydrolysate of lysozyme for use in the method for the treatment of impaired cognitive functioning in a subject in need thereof, wherein the subject is a human child, 18 years of age or younger, or wherein the subject is a human subject 18-65 years of age.

One embodiment is the subtilisin A hydrolysate of lysozyme for use in the method for the treatment of impaired cognitive functioning in a subject in need thereof, wherein the subject is male, preferably a human male, more preferably a human male at least 60 years of age, preferably at least 65 years of age, more preferably at least 67 years of age, such as 60-105 years of age or 67-75 years of age. As outlined here above especially elder men 67 years of age or older benefit above average from the daily intake of a composition comprising subtilisin A hydrolysate of lysozyme, when improvement of cognitive functioning is assessed.

According to embodiments of the invention, in the non-therapeutic method of improving cognitive functioning in a subject, at least 30% by weight of the subtilisin A hydrolysate of lysozyme are lysozyme peptides with a molecular weight of less than 0,5 kDa. Furthermore, for the subtilisin A hydrolysate of lysozyme for use in the method for the treatment of impaired cognitive functioning in a subject in need thereof, at least 30% by weight of the subtilisin A hydrolysate of lysozyme are lysozyme peptides with a molecular weight of less than 0,5 kDa.

Preferred is the non-therapeutic method of improving cognitive functioning in a subject, the method comprising administering to the subject a composition comprising subtilisin A hydrolysate of lysozyme, wherein the degree of hydrolysis of the lysozyme is at least 12%, preferably 15% - 50%, more preferably 18% - 40%, most preferably 19% - 30%, such as 21%. Also preferred is the subtilisin A hydrolysate of lysozyme for use in the method for the treatment of impaired cognitive functioning in a subject in need thereof, wherein the degree of hydrolysis of the lysozyme is at least 12%, preferably 15% - 50%, more preferably 18% - 40%, most preferably 19% - 30%, such as 21%.

According to the present invention, the composition comprises subtilisin A hydrolysate of lysozyme. Thus lysozyme is hydrolysed with subtilisin A. Subtilisin A is an endopeptidase, more specifically a serine protease even more specifically a subtilase. Subtilisin A is amongst others commercially marketed as Alcalase^{™}. The composition is for example a nutritional composition or a food supplement.

It is known in the art how to obtain a subtilisin A hydrolysate of lysozyme. A method for obtaining a subtilisin A hydrolysate of lysozyme is for example described in WO 2006/009448 and example 1.

Lysozyme is commercially available and may for example be obtained from BouwhuisEnthoven (100% pure protein). Lysozyme may also be extracted from eggs and subsequently purified. In one embodiment of the invention, the lysozyme that is subjected to subtilisin A hydrolysis is preferably purified lysozyme. The concentration of lysozyme in egg white is high (3-4%) as compared to other sources of lysozyme. A process used routinely for lysozyme purification is cation exchange chromatography. Lysozyme is bound to a cation exchanger at the pH as is (pH 9). This may be done in a stirred tank reactor or in a chromatography column. After elution from the adsorption particles by salt, lysozyme is pure enough for food applications. Anion exchange chromatography at pH 4 may be applied for further purification in order to obtain highly pure lysozyme, suitable for pharmaceutical applications. The advantages of this purification process are that the starting material (egg white) is not altered, the process can easily be up-scaled, for food applications just one adsorption process is needed and the biological activity is retained. Thus, in one embodiment according to the present invention, the lysozyme that is subjected to subtilisin A hydrolysis is purified using cation exchange chromatography.

The subtilisin A hydrolysate of lysozyme preferably comprises a high proportion of di- and/or tri peptides, preferably at least 10, 20, 30, 40, 50, 60, 70, wt.% or more of the weight of subtilisin A hydrolysate of lysozyme, more preferably at least 20 wt.%, even more preferably at least 30 wt.%, most preferably at least 40 wt.%. In one embodiment peptides of longer chain length and higher molecular weight, such as peptides with 4, 5, 6, 7, 8 or more amino acids, are therefore preferably present in amounts of less than 60 wt.% of the weight of subtilisin A hydrolysate of lysozyme.

In one embodiment, the subtilisin A hydrolysate of lysozyme preferably comprises peptides of less than 0.5 kD in a proportion of at least 25, 30, 40 or 45 wt.% of the weight of subtilisin A hydrolysate of lysozyme, preferably the subtilisin A hydrolysate of lysozyme comprises at least 30 wt.%, most preferably at least 40 wt.%. peptides of less than 0.5 kD. Preferably the subtilisin A hydrolysate of lysozyme comprises peptides of less than 0.5 kD in a proportion of at most 80, 70, 60 or 50 wt.% of the weight of subtilisin A hydrolysate of lysozyme.

In one embodiment, the subtilisin A hydrolysate of lysozyme comprises a distribution of fragments of molecular weight of < 0.5kD : from 0.5 to 1.0 kD : >1kD) in a ratio of 30-70 : 70-15 : 70-15. As an indication, fragments of less than 0.5 kD correspond to fragments of less than 4-5 amino acids, while fragments of about 0.5-1.0 kD correspond to 4-9 amino acids and fragments of above 1 kD correspond to peptide fragments of above 9 amino acids.

Preferably, the degree of hydrolysis of the subtilisin A hydrolysate of lysozyme is at least 15%, more preferably at least 20%, most preferably at least 30%. The degree of hydrolysis is preferably determined with the TNBS method, as for example described in WO 2006/009448. The degree of hydrolysis is the difference in the number of free amino groups in the hydrolysate and the number of free amino groups in the intact protein divided by the total amount of peptide bonds in the hydrolysate/intact protein. Generally the degree of hydrolysis is expressed in percentage, so the resultant value is multiplied by 100%.

A molecular weight distribution of the subtilisin A hydrolysate of lysozyme preferably comprises 30-70% target peptides of less than 0.5 kD and 15-70% peptides between 0.5 kD and 1 kD based of the weight of subtilisin A hydrolysate of lysozyme. The molecular weight distribution, peptide chain length distribution and maximum peptide weight of the hydrolysate can be determined by methods known in the art, such as SDS-PAGE analysis, HPLC analysis, MALDI-TOF (Matrix-Assisted Laser Desorption/lonisation Time-of-Flight mass spectrometry, as described by Kaufmann, J. Biotechn 1995, 41:155-175 and Soeryapranata et al. 2002, J. Food Sci. 67:534-538), HP-GPC (high performance gel permeation chromatrography, as described in Terheggen-Lagro et al. BMC Pediatrics 2002, 2:10) and Edman degradation (Siemensma et al. Trends Food Sci Technology 1993, 4:16-21). The maximum molecular peptide weight of the target protein is preferably less than 10 kD.

Alternatively a composition resembling a subtilisin A hydrolysate of lysozyme of this invention can be made *de novo* making use of chemical synthesis methods. In particular a di- and a tri-peptide library can be made by combinatorial chemistry thereby forming all possible combinations of dipeptides and tripeptides. From this pool, or library of di- and tripeptides a mixture can be composed having essentially the same activity on cognitive functioning as the hydrolysates described above.

The composition comprising subtilisin A hydrolysate of lysozyme preferably improves cognitive functioning or prevents a decline of cognitive functioning or ameliorates or reverses previously declined cognitive functioning, after regular, preferably daily, intake of an effective amount. An effective amount of the composition or effective dose in the context of the current invention is such an amount of the composition to obtain the claimed effect, e.g. a therapeutic effect or a prophylactic effect *in vivo.* The effective amount which needs to be administered to a subject depends on a number of factors, such as the subject (e.g. human or animal), the dosage form (daily, several times a day, weekly) and product composition and/or texture. It is within the realm of a skilled person to determine the effective amount using routine experimentation. Preferably, an effective amount is at least 0.1 grams of the claimed composition per day, more preferably at least 0.5 grams, even more preferably at least 1 grams, even more preferably at least 2 grams, most preferably at least 4 grams. For example, the effective amount is about 5 grams, such as 5,0 grams, administered to the subject for example once a day. Preferably an effective amount is at least 7 mg/kg body weight of the subject per day, more preferably at least 14 mg/kg, even more preferably at least 28 mg/kg, most preferably at least 56 mg/kg body weight of the subject per day. For example, the effective amount is between 40 mg/kg body weight of the subject per day and 100 mg/kg body weight of the subject per day, for example for subjects with a body weight of between 50 kg and 120 kg. Typically, a daily dose of the subtilisin A hydrolysates of lysozyme for a human subject is less than 10 gram, such as 0,5 - 9 gram/daily.

Provided is thus a composition comprising the subtilisin A hydrolysates of lysozyme, generated as described above or generated as described in Example 1, and optionally further enriched and/or purified for use according to the present invention. Also provided is a composition, especially a food product, a functional food product and/or a food supplement composition, comprising a suitable amount of a subtilisin A hydrolysates of lysozyme for use according to the present invention.

Food supplements and food products comprising at least one subtilisin A hydrolysate of lysozyme according to the invention may be made as known in the art. Food supplements may for example be in the form of any dosage form such as tablets, pills, powder sachets, gels, capsules, and the like. Intake by the subject is preferably oral. Food products may be in the form of drinks, solid or semi-solid foods, such as snacks, deserts, sauces, whole meals, *etc.* Preferably the food product is a product which is consumed on a regular basis, preferably daily, such as staple foods, e.g. bread, noodles, soft drinks, dairy products such as cheese, yoghurt, fermented dairy products, milk, butter, *etc.* The subtilisin A hydrolysates of lysozyme may thus be added to a food base or may be incorporated into the food product during its production process. Thus, any existing food or food supplement products comprising a subtilisin A hydrolysates of lysozyme for use according to the present invention are included herein.

In a preferred embodiment the food product is a drink, preferably based on a fruit juice or vegetable juice, although milk based drinks are also included. The drink may be made in daily dosage volumes of 50 ml, 100 ml, 150 ml, 200 ml or more. Preferred is an aqueous drink made in a daily dosage volume of 200 ml, for example comprising 5 grams of subtilisin A hydrolysates of lysozyme, or less than 10 gram such as 0,1 - 9 gram. It is understood that the food supplement or food product may further comprise additional food-grade ingredients, such as but not limited to flavourings, vitamins, minerals, stabilizers, emulsifiers, other biologically active ingredients, food bases/nutrients such as a protein, carbohydrate and/or fat component, and the like. The subtilisin A hydrolysate of lysozyme may be added at any stage during the normal production process of the food product/food supplement.

The food product / food supplement may also comprise other inactive ingredients and carriers, such as e.g. glucose, lactose, sucrose, mannitol, starch, cellulose or cellulose derivatives, e.g. carboxymethylcellulose (CMC), magnesium stearate, stearic acid, sodium saccharin, talcum, magnesium carbonate and the like. It may also comprise water, electrolytes, essential and non-essential amino acids, trace elements, minerals, fibre, sweeteners, flavourings, colorants, emulsifiers and stabilizers (such as soy lecithin, citric acid, esters of mono- or di-glycerides), preservatives, binders, fragrances, and the like.

For food supplements in the form of pills, tablets or capsules, a coating may be added which changes the place and/or time of release of the hydrolysate in vivo. Slow release formulations are known in the art.

The (food) product comprising subtilisin A hydrolysates of lysozyme or the subtilisin A hydrolysates of lysozyme may be ingested prophylactically by subjects at risk of a decline in cognitive functioning. The cognitive functioning is any of proactive inhibitory control and sustained attention in a subject. Furthermore, the proactive inhibitory control is for example control of impulsivity, and/or the sustained attention is for example general alertness.

In one embodiment, the composition according to the invention is an aqueous composition. The composition according to the invention may be a ready-for-use solution, as a stock solution from which the daily dose may be obtained or prepared by dilution or it may be a dry composition from which a daily dose may be obtained by adding a fluid. The composition may also be used as dry matter and mixed with a food stuff, The skilled person is well aware of these and other ways of administering a composition to a subject in need of the composition.

Preferably, the composition is ingested either as a food supplement, e.g. in the form of tablets, sachets, *etc.,* or as a functional food, e.g. in the form of drinks, semi-solid or solid food products.

For a preferred non-therapeutic method of improving cognitive functioning in a subject, the daily dose of the subtilisin A hydrolysate of lysozyme is 0,5 gram - 20 gram per day, preferably 1 gram - 10 gram per day, or less than 10 gram/daily, more preferably 2 gram - 8 gram per day, such as 5 gram per day. The daily dose is preferably administered to the subject as a single daily dose. Preferred is the subtilisin A hydrolysate of lysozyme for use in the method for the treatment of impaired cognitive functioning in a subject in need thereof, wherein the daily dose of the subtilisin A hydrolysate of lysozyme is 0,5 gram - 20 gram per day, preferably 1 gram - 10 gram per day, more preferably 2 gram - 8 gram per day, such as 5 gram per day. The daily dose is preferably administered to the subject as a single daily dose. For example, the single daily dose is administered to the subject as an aqueous drink of 50-300 ml, such as 100 ml, 125 ml, 200 ml, 250 ml, and a volume of about 200 ml comprising about 5 grams of subtilisin A hydrolysates of lysozyme is preferred.

An aspect of the current invention relates to the use of a nutraceutical, a food product, a functional food, a food stuff, a food ingredient, a food supplement, comprising a subtilisin A hydrolysate of lysozyme, in the non-therapeutic method of improving cognitive functioning in a subject according to the invention. The subtilisin A hydrolysate of lysozyme is the hydrolysate of any of the aforementioned aspects and embodiments of the invention.

An aspect of the current invention relates to a nutraceutical, a food product, a functional food, a food stuff, a food ingredient, a food supplement and/or a feed product, comprising a subtilisin A hydrolysate of lysozyme, for use in the method for the treatment of impaired cognitive functioning in a subject in need thereof according to the claims. The subtilisin A hydrolysate of lysozyme is the hydrolysate of any of the aforementioned aspects and embodiments of the invention.

To the surprise of the inventors, they are the first to establish that daily consumption of less than 10 gram of the subtilisin A hydrolysates of lysozyme for at least 4 weeks results in improved cognition, wherein the improved cognition is e.g. improved control of impulsivity, improved inhibitory control, or the combination of improved control of impulsivity, improved inhibitory control, improved alertness and improved attention, not previously established in the prior art to the knowledge of the inventors.

The invention is further illustrated by the following examples, which should not be interpreted as limiting the present invention in any way.

### EXAMPLES

### Example 1: Preparation of a subtilisin A hydrolysate of lysozyme.

SDS-PAGE (determination of molecular weight): The molecular weight of the peptides was determined by SDS-PAGE. Samples and a molecular weight marker (10-250 kDa) were applied to a 4-20% Criterion TGX gel. Subsequently, a voltage was applied over the gel and after migration of the samples, the gel was stained by Coomassie Blue.

Bulk density: Bulk density was measured in a 50 mL graduated cylinder. The cylinder was filled with 50 mL powder and weighed. The cylinder was tapped once. The bulk density is expressed as g powder/mL.

Molecular size distribution (Size Exclusion Chromatography (SEC)): Samples were injected on a Superdex peptide column. Potassium phosphate buffer and sodium sulphate buffer in water was used as eluent. Detection was performed by refractive index and UV 220 nm. A mixture of peptides and maltoses was used to calibrate the column.

A 5% (w/v) solution of lysozyme in water (100% protein content, BouwhuisEnthoven, Raalte, The Netherlands) was prepared and adjusted to a pH between 7.5 and 8.5 with 3 M KOH. Hydrolysis was started by adding Alcalase^{™} (Novozymes) to a final concentration of 4% on protein basis. The solution was incubated for a total of 6 hours at 60°C, under continuous stirring. Alcalase ^{™} was then inactivated by increasing the temperature to 90°C for 15 minutes. The solution was then cooled down to 2°C and stored overnight under continuous stirring.

The resulting hydrolysate solution was filtered through a 10 µm filter and subsequently through a 1 µm filter. Thereafter, the filtrate was heat treated for 15 s at 135°C and concentrated to a dry matter of 57 °Brix (approximately dry matter of 45%) by a NIRO evaporator at a flow of 3300 L/h at 90°C. After evaporation, the product was spray dried to obtain a powder with very good flowability properties, as evidenced by visual observation.

The final product had the following characteristics: white powder, good solubility, degree of hydrolysis of 21% (TNBS method) and a maximum molecular weight of less than 10 kDa. Peptide size distribution was as follows: 46% <500Da, 23% 500-1000 Da, 32% >1000 Da.

Similarly, a 5% (w/v) solution of lysozyme in water (100% protein content, BouwhuisEnthoven, Raalte, The Netherlands) was prepared and adjusted to a pH of 8,0 with 3 M KOH. Hydrolysis was started by adding Alcalase^{™} (Novozymes) to a final concentration of 4% on protein basis. The solution was incubated for a total of 5 hours at 60°C, under continuous stirring. Alcalase^{™} was then inactivated by increasing the temperature to 90°C for 15 minutes. The solution was then cooled down to 6°C and stored overnight under continuous stirring.

The resulting hydrolysate solution was filtered through a 100 µm filter and subsequently through a 1 µm filter. Thereafter, the filtrate was heat treated for 15 s at 135°C and concentrated to a dry matter of 56,5 °Brix (approximately dry matter of 45%) by a NIRO evaporator at a flow of 3300 L/h at 90°C. After evaporation, the product was spray dried to obtain a powder with very good flowability properties, as evidenced by visual observation. The particle size distribution was assessed and showed that the majority of the particles on a log scale had a Gaussian distribution around 120 micrometer (between 40 micrometer and 400 micrometer) and a relatively small portion of particles was larger than 500 micrometer. Size distribution: d(0,1) was 68 micrometer in the end product, d(0,5) was 124 micrometer, d(0,9) was 242 micrometer. Particle size distribution of the powder was measured by a laser diffraction technique, using a Mastersizer 2000 (Malvern). Powder samples were dispersed in isopropanol before measurements. Bulk density of the obtained white hydrolysate powder was 0,42 g/mL.

The final product had the following characteristics: white powder, good solubility, degree of hydrolysis of 21% and a maximum molecular weight of less than 10 kDa. Peptide size distribution was as follows: 46% <500Da, 23% 500-1000 Da, 32% >1000 Da. Degree of hydrolysis: The degree of hydrolysis (DH) was determined by the orthophthaldialdehyde (OPA) method. The amount of free amino groups was determined by staining with orthophthaldialdehyde and spectrophotometric detection at 412 nm. The DH is defined as: [((# total free amino groups) - (# free amino groups in intact protein)) * 100%] / [Total amount of peptide bonds]. Dry matter content was 97,90% (moisture content was 2,10%). Dry matter (DM): The dry matter content was measured by an infrared moisture analyser (Sartorius). A sample of 2 mL was distributed over a filter paper and weighed. Subsequently, the samples were dried until a constant weight was reached. DM = (weight after drying)/(weight before drying)*100%.

### Example 2: Clinical study

The study concerned is a randomized, double-blind, placebo controlled, cross-over clinical trial. In Fig. 1, an outline of the clinical trial protocol is shown. The objective of the study is towards the 4-weeks effect of a composition comprising subtilisin A hydrolysate of lysozyme on cognitive functioning, more in particular on improvement of inhibitory control and improvement of attention in a subject, such as proactive inhibitory control and/or sustained attention, more in particular wherein the proactive inhibitory control is control of impulsivity, and/or wherein the sustained attention is alertness, such as general alertness. Tests for assessing cognitive functioning were done preceeding the 4-weeks period in which the composition comprising subtilisin A hydrolysate of lysozyme or placebo were daily administered to the subjects (time point V3 and V6 in Fig. 1), and again at the end of the 4-weeks period (time point V5 and V8 in Fig. 1). All measurements were performed in otherwise healthy subjects yet meeting the criteria to be diagnosed with metabolic syndrome. Each 4-weeks intervention period was separated by a wash-out period of typically four weeks, but it was allowed to extend or shorten this period such that the wash-out period was between 2 and 8 weeks.

The composition comprising subtilisin A hydrolysate of lysozyme or the placebo was provided as separate portions of 5 g in sachets as a white powder that had good solubility. Subjects were asked to dissolve the powder in approximately 200 mL of water prior to consumption. Placebo was also provided as a powder that was similar to the hydrolysate in colour and taste. Sixty-eight subjects were randomized at the start of the study.

A first cognition functioning test performed was a sustained attention test (SAT), a vigilance task designed to assess the ability of individuals to sustain attention, used for assessing reaction time and used for assessing attention. The SAT is a psychomotor vigilance test, also used for assessing sustained attention and for assessing general alertness. For the SAT, the preparation intervals were 1-2, 3-4, 5-6, 7-8 and 9-10 s. The psychomotor vigilance test is a simple task to evaluate sustained attention. The subjects were instructed to press a button as soon as a stimulus appeared. The stimulus consisted of a square in the middle of the screen turning green. In this study, the duration of a single trial comprised 8 minutes. During this 8 minutes, 80 stimuli appeared and the interstimulus interval varied randomly between 1 and 10 seconds. The first time subjects had to perform the test, four demo trials were used to show the principle of the test. After the demo trials, eight practise trials had to be performed, followed by eighty official test trials.

A second cognition test performed was the anticue task, a 4-choice reaction time (RT) task, with left and right anticues indicating mirror-symmetrical response hands. In particular anticues require participants to prepare fingers on the hand opposite to the side of the cue (counter-corresponding mapping). For the outline of the anticue task test reference is made to "Switch hands! Mapping temporal dynamics of proactive manual control with anticues", J.J. Adam, S. Jennings, T.J.H. Bovend'Eerdt, P.P.M. Hurks, P.W.M. Van Gerven, Acta Psychologica 161 (2015), pp.137-144. The anticue task assesses proactive cognitive control, especially relating to impulse control. The anticue task examines the efficiency and time course of proactive control operations, wherein the anticue task focuses on the resolution of response conflict in the manual motor system. The anticue test thus assesses impulse control, reaction time and executive function. In the current clinical trial the anticue set-up was used ('uncued anticue test') and the anticue:cue set-up was used ('cued anticue test'). For both set-ups improvement in reaction time was assessed under unfluence of the intake of the lysozyme hydrolysate ('Treatment' in Fig. 1) or placebo according to the invention. Fixation time was about 750 msec, gap time was about 750 msec, and the preparation intervals (target intervals) were 100 msec, 150 msec, 250 msec, 450 msec or 850 msec.

Statistical analysis: cognition test data have been analyzed via a dummy stepwise paired General Estimating Equations procedure with identification as link, in which change values were treated as dependent parameter while gender, age, body mass index, following order of consumption of product, treatment, preparation/target intervals, interaction of intervals and treatment, and value at first preparation interval have been applied as independent parameters. Treatment was also evaluated as independent parameter without addition of the other parameters. Fit of the model was evaluated via Wald Chi square test. Outliers were identified via Grubbs test. Since no missing values were recorded and no drop-outs were observed, Intention-to-Treat (ITT) population with and without outliers have been analyzed. Throughout the study a p-value of 0.05 was used to identify significance, applying two-sided evaluation. Statistical analysis was performed using STATA, version 12 (StataCorp, College Station, TX, US) and GraphPad, version 6 (GraphPad Prism, LaJolla, CA, US).

### STUDY POPULATION

### POPULATION BASE

The study population consisted of otherwise healthy males and female subjects, aged between 18 and 75 years, and meeting the criteria for Metabolic Syndrome. Subjects were non-smokers with a stable body weight.

Inclusion Criteria in order for clinical trial participants to be considered eligible for enrolment into the study, and which were met by the subjects enrolled in the clinical trial, were:
- Be able to give written informed consent,
- Be between 18 and 75 years of age,
- Be in generally good health as determined by the investigator,
- Be non-smokers
- Have a stable body weight (< 5% change) in the 3 months prior to study entry,
- Meet the harmonized criteria for the presence of Metabolic Syndrome as agreed by the International Diabetes Federation (IDF), National Heart Lung and Blood Institute, American Heart Association, World Heart Federation, International Atherosclerosis Society and International Association for the Study of Obesity, and defined as having at least three of the five following risk factors:
   ∘ Central obesity (waist circumference >94cms in males or > 80cms in females) or having a BMI > 30 kg/m
   ∘ Raised triglycerides (>1.7 mmol/L (150mg/dL)
   ∘ Reduced HDL cholesterol [<1.03mmol/L (40mg/dL) in males, <1.29mmol/L (50mg/dL) in females]
   ∘ Raised fasting plasma glucose > 5.6mmol/L (100mg/dL)
   ∘ Raised blood pressure (systolic blood pressure > 130 mmHg or diastolic blood pressure 85 mmHg)

See also Table 1 for the clinical trial subject characteristics.

### Exclusion criteria

Presented human subjects were excluded from the study if they met any of the below criteria;
1. Are less than 18 years of age or over 75 years of age,
2. Females who are pregnant, breast feeding or who may wish to become pregnant during the study,
3. Are hypersensitive to any of the components of the test product (i.e. egg protein),
4. Have a significant acute or chronic coexisting illness such as cardiovascular disease, chronic kidney disease (CKD), gastrointestinal disorder, endocrinological disorder, immunological disorder, metabolic disease or any condition which contraindicates, in the investigators judgement, entry to the study,
5. Having a condition or have taken a medication that the investigator believes would interfere with the objectives of the study, pose a safety risk or confound the interpretation of the study results; to include diuretics, blood pressure lowering medication and medication otherwise interfering with renin-angiotensin-aldosterone system (RAAS), such as ACE-inhibitors, angiotensin receptor blockers, direct renin inhibitors or aldosterone receptor inhibitor and cholesterol lowering agents such as statins.
6. Are taking non-steroidal anti-inflammatory drugs (NSAIDs) within 2 weeks of baseline visit or for the duration of the trial,
7. Suffer from diabetes mellitus, either type I and type II,
8. Consume more than the recommended alcohol guidelines i.e. >21 alcohol units/week for males and >14 units/week for females,
9. History of illicit drug use,
10. Individuals who, in the opinion of the investigator, are considered to be poor attendees or unlikely for any reason to be able to comply with the trial,
11. Subjects may not be receiving treatment involving experimental drugs,
12. If the subject has been in a recent experimental trial, these must have been completed not less than 30 days prior to this study or if the subject has donated blood, at a blood bank, within period of 8 weeks prior to the start of the study.
13. Have a malignant disease or any concomitant end-stage organ disease.

The subtilisin A hydrolysate of lysozyme as the active ingredient has been produced by NIZO Food Research in Ede, The Netherlands, enabling the spray-dried product to be manufactured and tested. Ingredients of the active sachet: The subtilisin A hydrolysate of lysozyme as the active ingredient, citric acid, flavouring, Acesulfame K, Sucralose, Quinine HCL. Ingredients of the placebo sachet: Maltodextrin, flavouring, citric acid, cloudifier, tartaric acid, Malic Acid, Acesulfame K, Sucralose, Caramel E150a, Quinine HCL. Each sachet was dissolved in approximately 200 mL of water prior to consumption. Placebo sachets contain maltodextrin of potato starch as inert filling material instead of the hydrolysate. The active ingredient comprising powder and the placebo powder were similar in colour, and taste. The taste of both products was bitter lemon. Sachets were manufactured according to generally accepted procedures and Good Manufacturing Practices.

### Results of the SAT

All 68 clinical trial participants were assessed as a single group of subjects. See Tables 2-4 for measured values. A decrease in reaction time values between interval 1 (1-2 sec) and 2 (3-4 sec) is observed, and the measured reaction time per study arm remains about constant between interval 2 and 3 (5-6 sec), 3 and 4 (7-8 sec), and 4 and 5 (9-10 sec). The measured test results showed that the change in reaction time is significantly larger (less time needed after 4 weeks) in the treatment group (subjects were administered the lysoszyme hydrolysate) than in the placebo group of subjects.

Comparing the test results determined at day 0 with the test results determined after 4 weeks of treatment, for the five preparation intervals the reaction time after 4 weeks of treatment was 5-10 msec shorter for the subjects who received the lysoszyme hydrolysate of the invention compared to the subjects who received placebo. This shortened reaction time is an improvement of about 1-3%, or about 1,3-2,7%. Also the change in reaction time was significantly larger (less time needed after 4 weeks of treatment) in the group of subjects to whom lysosyzme lysate was administered, compared to placebo group. Thus, the daily intake of the composition comprising subtilisin A hydrolysate of lysozyme results in a larger reduction of the time required to fulfil the tasks of the SAT, as compared to the daily consumption of placebo.

| Table 1: characteristics of the human subjects participating in the clinical trial | | | | | | | | |
|---|---|---|---|---|---|---|---|---|
| Gender (1, male; 2, female) | Age (years) | Age category | BMI | | Gender (1, male; 2, female) | Age (years) | Age category | BMI |
| 1 | 36 | 1 | 29,5 | | 1 | 51 | 1 | 30 |
| 1 | 72 | 3 | 30,6 | | 1 | 66 | 2 | 31,1 |
| 1 | 59 | 2 | 29,1 | | 1 | 49 | 1 | 27,7 |
| 1 | 68 | 3 | 31,4 | | 1 | 57 | 2 | 30,5 |
| 2 | 46 | 1 | 37 | | 1 | 53 | 1 | 26,5 |
| 1 | 62 | 2 | 28,9 | | 2 | 63 | 2 | 28,8 |
| 2 | 65 | 2 | 32,2 | | 2 | 56 | 2 | 30,7 |
| 2 | 44 | 1 | 33,4 | | 2 | 41 | 1 | 41 |
| 2 | 68 | 3 | 28,9 | | 2 | 52 | 1 | 25,8 |
| 2 | 53 | 1 | 36,4 | | 2 | 57 | 2 | 36,2 |
| 1 | 61 | 2 | 36,8 | | 1 | 66 | 2 | 26,3 |
| 1 | 67 | 3 | 28,8 | | 2 | 72 | 3 | 27 |
| 1 | 72 | 3 | 32,2 | | 1 | 71 | 3 | 34,2 |
| 1 | 50 | 1 | 35,3 | | 2 | 41 | 1 | 28,7 |
| 1 | 51 | 1 | 26,6 | | 2 | 51 | 1 | 36,2 |
| 1 | 52 | 1 | 26 | | 2 | 75 | 3 | 26,6 |
| 1 | 75 | 3 | 31,2 | | 1 | 71 | 3 | 36,6 |
| 2 | 68 | 3 | 31,1 | | 1 | 68 | 3 | 27,4 |
| 1 | 69 | 3 | 31,2 | | 2 | 43 | 1 | 29,7 |
| 1 | 60 | 2 | 37,2 | | 2 | 61 | 2 | 28,7 |
| 1 | 55 | 1 | 36,9 | | 1 | 68 | 3 | 30,4 |
| 2 | 75 | 3 | 37,1 | | 2 | 68 | 3 | 39,8 |
| 1 | 66 | 2 | 30,4 | | 2 | 64 | 2 | 32,3 |
| 2 | 74 | 3 | 33,5 | | 1 | 71 | 3 | 30,2 |
| 1 | 71 | 3 | 36,8 | | 2 | 73 | 3 | 35,1 |
| 2 | 35 | 1 | 36,2 | | 1 | 65 | 2 | 34,1 |
| 1 | 67 | 3 | 28,2 | | 2 | 40 | 1 | 35,4 |
| 2 | 57 | 2 | 30,3 | | 2 | 61 | 2 | 26,1 |
| 1 | 50 | 1 | 35,4 | | 1 | 71 | 3 | 33,5 |
| 1 | 50 | 1 | 29,2 | | 2 | 63 | 2 | 24,9 |
| 1 | 53 | 1 | 33,7 | | 2 | 69 | 3 | 30,9 |
| 2 | 64 | 2 | 35,2 | | 1 | 63 | 2 | 41,9 |
| 1 | 62 | 2 | 35,1 | | 2 | 67 | 3 | 27 |
| 1 | 63 | 2 | 26 | | 1 | 70 | 3 | 27,4 |

**TABLE 2: Descriptive values of reaction time at the various preparation intervals, numbered as 1, 2, 3, 4 and 5, corresponding to intervals of 1-2 sec, 3-4 sec, 5-6 sec, 7-8 sec, and 9-10 sec, respectively, as observed in the SAT.**

| Placebo | | | | | | | | | | |
|---|---|---|---|---|---|---|---|---|---|---|
| | placebo 0 | | | | | placebo 27 | | | | |
| | 1 | 2 | 3 | 4 | 5 | 1 | 2 | 3 | 4 | 5 |
| mean | 406.8 | 362.1 | 359.0 | 358.4 | 360.6 | 411.5 | 376.3 | 375.1 | 371.6 | 372.5 |
| stdev | 102.7 | 72.2 | 66.8 | 68.6 | 71.7 | 78.1 | 69.0 | 76.7 | 71.8 | 65.7 |
| N | 68 | 68 | 68 | 68 | 68 | 68 | 68 | 68 | 68 | 68 |
| s.e.m. | 12.5 | 8.8 | 8.1 | 8.3 | 8.7 | 9.5 | 8.4 | 9.3 | 8.7 | 8.0 |

### Treatment

| | treatment 0 | | | | | treatment 27 | | | | |
|---|---|---|---|---|---|---|---|---|---|---|
| | 1 | 2 | 3 | 4 | 5 | 1 | 2 | 3 | 4 | 5 |
| mean | 398.8 | 371.6 | 360.4 | 364.0 | 366.9 | 413.2 | 376.0 | 367.7 | 371.2 | 372.8 |
| stdev | 75.6 | 68.8 | 63.5 | 75.9 | 67.4 | 94.9 | 74.6 | 70.6 | 80.3 | 71.1 |
| N | 68 | 68 | 68 | 68 | 68 | 68 | 68 | 68 | 68 | 68 |
| s.e.m. | 9.2 | 8.3 | 7.7 | 9.2 | 8.2 | 11.5 | 9.0 | 8.6 | 9.7 | 8.6 |

**TABLE 3: Descriptive values of the change in reaction time between day 0 and after 4 weeks, at the five preparation intervals, numbered as 1, 2, 3, 4 and 5, as observed in the SAT.**

| changes | Placebo | | | | | | treatment | | | | |
|---|---|---|---|---|---|---|---|---|---|---|---|
| | 1 | 2 | 3 | 4 | 5 | | 1 | 2 | 3 | 4 | 5 |
| mean | 4.71 | 14.25 | 16.16 | 13.24 | 11.93 | | 14.40 | 4.41 | 7.29 | 7.18 | 5.91 |
| stdev | 78.77 | 45.19 | 50.78 | 48.16 | 55.69 | | 63.17 | 46.32 | 41.75 | 46.59 | 46.34 |
| N | 68 | 68 | 68 | 68 | 68 | | 68 | 68 | 68 | 68 | 68 |
| s.e.m. | 9.55 | 5.48 | 6.16 | 5.84 | 6.75 | | 7.66 | 5.62 | 5.06 | 5.65 | 5.62 |

**TABLE 4: Descriptive values of the change in reaction between day 0 and after 4 weeks, at the five preparation intervals, numbered as 1, 2, 3, 4 and 5, without outliers (for each preparation interval data is included for at least 65 subjects), as observed in the SAT.**

| changes | placebo | | | | | | treatment | | | | |
|---|---|---|---|---|---|---|---|---|---|---|---|
| | 1 | 2 | 3 | 4 | 5 | | 1 | 2 | 3 | 4 | 5 |
| mean | 15.50 | 17.64 | 13.89 | 14.29 | 11.92 | | 11.02 | 3.91 | 9.16 | 9.77 | 4.16 |
| stdev | 48.38 | 35.76 | 37.06 | 40.10 | 34.81 | | 54.49 | 39.78 | 39.09 | 36.56 | 44.37 |
| N | 66 | 67 | 65 | 66 | 66 | | 66 | 66 | 67 | 65 | 67 |
| s.e.m. | 5.96 | 4.37 | 4.60 | 4.94 | 4.29 | | 6.71 | 4.90 | 4.78 | 4.54 | 5.42 |

### Results of the uncued anticue test

The change in reaction time (msec) between day 0 (time point V3 and V6 in Fig. 1) and at 4 weeks (end of intervention period; time point V5 and V8 in Fig. 1) at the five target intervals 100 msec., 150 msec., 250 msec., 450 msec. and 50 msec, was assessed for the group of healthy subjects to whom placebo was administered daily for four weeks, and for the group of healthy subjects to whom the composition comprising subtilisin A hydrolysate of lysozyme was administered ('treatment' in Fig. 1). See Tables 5-7 for the data sets relating to the uncued anticue test.

The administration of placebo for four weeks did not have an effect on the reaction time, when the five target intervals are assessed. The intake of placebo for four weeks therefore did not influence performance of the human subjects in the uncued anticue test. In contrast, intake of the composition comprising subtilisin A hydrolysate of lysozyme of the invention by the treatment group of subjects resulted in a decrease of the required reaction time for performing the task. The improvement of reaction time under influence of intake of the lysozyme hydrolysate was about the same for the five target intervals assessed, and was about 10,4 msec. This decreased reaction time is an improvement of about 2-3%. When the five different target intervals are considered, the difference in the change values between day 0 and after 4 weeks of treatment between placebo group of subjects and treatment group of subjects remains at a constant rate (p < 0,009). When all participants in the clinical trial were assessed (male, female), it was observed that the improvement of the reaction time was negatively correlated with age: the older the subject, the lower the decrease in reaction time (p < 0,001).

Results obtained with left hands and right hands are combined, before calculating average interaction times and the difference in reaction times between day 27 ('d27') and day 0 ('d0').

**TABLE 5: Descriptive values of the change in reaction time at the applied target intervals in the uncued anticue test, numbered as 100, 150, 250, 450 and 850 msec.**

| | **Placebo 0 days** | | | | | | **Placebo 27 days** | | | | |
|---|---|---|---|---|---|---|---|---|---|---|---|
| *msec.* | *100* | *150* | *250* | *450* | *850* | | *100* | *150* | *250* | *450* | *850* |
| mean | 0 | -9.54 | -23.64 | -20.34 | -9.95 | | 0.00 | -8.29 | -21.41 | -19.38 | -8.44 |
| stdev | 0 | 28.53 | 30.41 | 29.75 | 32.43 | | 0.00 | 28.58 | 26.75 | 33.51 | 36.74 |
| N | 68 | 68 | 68 | 68 | 68 | | 68 | 68 | 68 | 68 | 68 |
| s.e.m. | 0 | 3.46 | 3.69 | 3.61 | 3.93 | | 0.00 | 3.47 | 3.24 | 4.06 | 4.46 |

| | **Treatment 0 days** | | | | | | **Treatment 27 days** | | | | |
|---|---|---|---|---|---|---|---|---|---|---|---|
| *msec.* | *100* | *150* | *250* | *450* | *850* | | *100* | *150* | *250* | *450* | *850* |
| mean | 0 | -16.57 | -18.70 | -19.21 | -5.00 | | 0.00 | -8.78 | -21.36 | -12.83 | 2.14 |
| stdev | 0 | 19.88 | 25.58 | 28.37 | 34.03 | | 0.00 | 23.35 | 26.74 | 33.14 | 30.41 |
| N | 68 | 68.00 | 68.00 | 68.00 | 68.00 | | 68.00 | 68.00 | 68.00 | 68.00 | 68.00 |
| s.e.m. | 0 | 2.41 | 3.10 | 3.44 | 4.13 | | 0.00 | 2.83 | 3.24 | 4.02 | 3.69 |

**TABLE 6: Descriptive values of the change in reaction time between day 0 and after 4 weeks of intake of either placebo or treatment (lysoszyme hydrolysate) at the applied target intervals in the uncued anticue test, numbered as 100, 150, 250, 450 and 850 msec.**

| | **Placebo** | | | | | | **Treatment** | | | | |
|---|---|---|---|---|---|---|---|---|---|---|---|
| *mseconds* | *100* | *150* | *250* | *450* | *850* | | *100* | *150* | *250* | *450* | *850* |
| mean | -2.91 | -1.67 | -0.68 | -1.96 | -1.40 | | -14.08 | -6.29 | -16.74 | -7.71 | -6.94 |
| stdev | 53.51 | 44.33 | 53.67 | 47.05 | 52.19 | | 40.75 | 45.83 | 50.23 | 42.69 | 43.20 |
| N | 68 | 68 | 68 | 68 | 68 | | 68.00 | 68.00 | 68.00 | 68.00 | 68.00 |
| s.e.m. | 6.49 | 5.38 | 6.51 | 5.71 | 6.33 | | 4.94 | 5.56 | 6.09 | 5.18 | 5.24 |

**TABLE 7: Descriptive values of the change in reaction time between day 0 and after 4 weeks of intake of either placebo or treatment (lysoszyme hydrolysate) at the selected target intervals in the uncued anticue test, numbered as 100, 150, 250, 450 and 850 msec, without outliers (for each reaction time data is included for at least 63 subjects).**

| | **Placebo** | | | | | | **Treatment** | | | | |
|---|---|---|---|---|---|---|---|---|---|---|---|
| *mseconds* | *100* | *150* | *250* | *450* | *850* | | *100* | *150* | *250* | *450* | *850* |
| mean | -3.03 | 1.34 | -1.55 | 1.91 | -1.4 | | -12.95 | -4.74 | -12.03 | -7.3 | -6.21 |
| stdev | 40.31 | 35.05 | 44.67 | 36.16 | 34.03 | | 40.22 | 42.35 | 38.63 | 42.09 | 36.46 |
| N | 63 | 64 | 65 | 64 | 63 | | 66 | 65 | 64 | 66 | 65 |
| s.e.m. | 5.08 | 4.38 | 5.54 | 4.52 | 4.29 | | 4.95 | 5.25 | 4.83 | 5.18 | 4.52 |

### Results of the cued anticue test

The change in reaction time (msec) between day 0 (time point V3, V6 in Fig. 1) and at 4 weeks (end of intervention period; time point V5, V8 in Fig. 1) at the five target intervals 100 msec., 150 msec., 250 msec., 450 msec. and 50 msec, was assessed for the group of healthy subjects to whom placebo was administered daily for four weeks, and for the group of healthy subjects to whom the composition comprising subtilisin A hydrolysate of lysozyme was administered ('treatment' in Fig. 1). Measured data points are summarized in Table 8 and Table 9. The administration of placebo for four weeks did have a negative effect on the reaction time, when the five target intervals are assessed. That is to say, for the five target intervals, after 4 weeks of treatment with placebo, reaction time was 0-7 msec slower when compared to the reaction time at day 0, before treatment with placebo started. The intake of placebo for four weeks therefore did not positively influence performance of the human subjects in the cued anticue test. In contrast, intake of the composition comprising subtilisin A hydrolysate of lysozyme of the invention by the treatment group of subjects for 4 weeks resulted in a decrease of the required reaction time for performing the task, when all five target intervals are assessed.

The improvement (i.e. decrease) of reaction time under influence of intake of the lysozyme hydrolysate was about the same for the first four target intervals assessed, and was about 12 msec (between 11,5 msec and 12,7 msec). For the target interval of 850 msec, the improvement of the reaction time after four weeks was about 2,6 msec compared to the reaction time at day 0. On average the improvement of the reaction time after four weeks was about 10 msec compared to the reaction time at day 0, four all five durations of the preparation interval combined. This decreased reaction time is an improvement of about 2-3%.

Thus, intake of the lysozyme hydrolysate for 4 weeks shortens the reaction time required for the test, when compared to intake of placebo, which appears to increase required reaction time to a certain extent. When the five different target intervals are considered, the difference in the change values between day 0 and after 4 weeks of treatment between placebo group of subjects and treatment group of subjects remains at a constant rate (p < 0,009). When all participants in the clinical trial were assessed (male, female), it was observed that the improvement of the reaction time was negatively correlated with age: the older the subject, the lower the decrease in reaction time (p < 0,001).

When human male of 67 years of age or older (67-75 years of age) are considered (14 males), improvement in reaction time (i.e. shorter reaction time) was larger compared to the improvement in reaction time when all 68 subjects were considered, for the reaction time assessed after intake of the lysoszyme hydrolysate for 4 weeks and compared to the reaction time at day 0 before the start of the 4-weeks intake. That is to say, the improvement compared to placebo was on average about 32 msec and varied between 22 msec and 40 msec when the five target intervals are assessed. For the 68 subjects assessed as a whole in a single group, the improvement compared to placebo was on average about 13 msec and varied between 9 msec and 16 msec when the five target intervals are assessed. Thus, relatively older male subjects benefit more from treatment with the lysozyme hydrolysate when improvement in cognitive function is considered, compared to younger males and females of all ages included in the clinical trial.

Results obtained with left hands and right hands are combined, before calculating average interaction times and the difference in reaction times between day 27 ('d27') and day 0 ('d0').

The improvement of the reaction time in the cued anticue test in terms of the reaction time becoming about 10 msec shorter under influence of intake of the composition comprising subtilisin A hydrolysate of lysozyme of the invention, is in absolute terms comparable to the increase in reaction time seen in human subjects over the course of between about one and two years. Thus, intake of the lysozyme hydrolysate, i.e. the subtilisin A hydrolysate of lysozyme of the invention, has an effect comparable to halting (natural) deterioration of cognitive function occurring during one to two years of increasing age.

**TABLE 8: Descriptive values of the change in reaction time between day 0 and after 4 weeks of intake of either placebo or treatment (lysoszyme hydrolysate) at the selected target intervals, numbered as 100, 150, 250, 450 and 850 msec, as determined in the cued anticue test.**

| | **Placebo** | | | | | | **Treatment** | | | | |
|---|---|---|---|---|---|---|---|---|---|---|---|
| *mseconds* | *100* | *150* | *250* | *450* | *850* | | *100* | *150* | *250* | *450* | *850* |
| mean | 3.16 | 1.25 | -0.13 | 4.75 | 6.98 | | -12.40 | -11.46 | -12.65 | -11.53 | -2.63 |
| stdev | 47.40 | 36.97 | 45.39 | 53.57 | 46.10 | | 42.47 | 44.53 | 43.56 | 46.29 | 47.46 |
| N | 68 | 68 | 68 | 68 | 68 | | 68 | 68 | 68 | 68 | 68 |
| s.e.m. | 5.75 | 4.48 | 5.50 | 6.50 | 5.59 | | 5.15 | 5.40 | 5.28 | 5.61 | 5.76 |

**TABLE 9: Descriptive values of the change in reaction time between day 0 and after 4 weeks of intake of either placebo or treatment (lysoszyme hydrolysate) at the selected target intervals in the cued anticue test, numbered as 100, 150, 250, 450 and 850 msec, without outliers (for each reaction time data is included for at least 63 subjects).**

| | **Placebo** | | | | | | **Treatment** | | | | |
|---|---|---|---|---|---|---|---|---|---|---|---|
| *mseconds* | *100* | *150* | *250* | *450* | *850* | | *100* | *150* | *250* | *450* | *850* |
| mean | 1.93 | -1.43 | -2.74 | 5.40 | 4.59 | | -9.58 | -11.90 | -9.24 | -5.50 | -0.49 |
| stdev | 42.26 | 31.59 | 37.21 | 34.48 | 31.90 | | 35.77 | 43.08 | 35.18 | 37.33 | 38.81 |
| N | 65 | 65 | 63 | 64 | 64 | | 65 | 66 | 65 | 64 | 65 |
| s.e.m. | 5.24 | 3.92 | 4.69 | 4.31 | 3.99 | | 4.44 | 5.30 | 4.36 | 4.67 | 4.81 |

### REFERENCES

1. Deacon, R., "Assessing burrowing, nest construction, and hoarding in mice", Journal of Visualized Experiments, January 2012, V59, e2607, pp.1-12.
2. Jirkof, P., "Burrowing and nest building behavior as indicators of well-being in mice", Journal of Neuroscience Methods, 2014, V234, pp.139-146.
3. Deacon, R.M.J., "Burrowing: A sensitive behavioural assay, tested in five species of laboratory rodents", Behavioural Brain Research, 2009, V200, pp.128-133.
4. Mackworth, N.H., "The breakdown of vigilance during prolonged visual search", Quarterly Journal of Experimental Psychology, 1948, V1, pp.6-21.
5. Jex, H.R., McDonnell, J.D., and Phatak, A.V., "A "critical" tracking task for man-machine research related to the operator's effective delay time", NASA contractor report NASA CR-616, November 1966, pp.1-105.
6. Petzoldt, T., Bellem, H., and Krems, J.F., "The critical tracking task - A potentially useful method to assess driver distraction?", Human Factors, 2014, V56(4), pp.784-803.

## Claims

1. Subtilisin A hydrolysate of lysozyme for use in a method for the prophylaxis or treatment any one or more of impaired inhibitory control and impaired control of impulsivity in a subject suffering from any one or more of a neurodegenerative disease, Alzheimer's disease, dementia, Parkinson's Disease, obsessive-compulsive disorder, compulsive neurosis, Tourette's syndrome, attention deficit hyperactivity disorder, a psychiatric disorder, Trichotillomania, schizophrenia, and an addiction to any one of a drug, a narcotics, nicotine, alcohol, gaming, and gambling.

2. Subtilisin A hydrolysate of lysozyme for use in the method of claim 1, wherein said use comprises reversing previously declined cognitive function of said subject.

3. Subtilisin A hydrolysate of lysozyme for use in the method of claim 1, wherein said use comprises amelioration or reversal of previously declined cognitive function.

4. Subtilisin A hydrolysate of lysozyme for use in the method of any one of claims 1 - 3, wherein said subject has at least one or more of declined proactive inhibitory control and impaired control of impulsivity.

5. Subtilisin A hydrolysate of lysozyme for use in the method of any one of claims 1 - 4, wherein the subject is a human of at least 50 years of age, preferably at least 60 years of age, more preferably at least 60 years of age, most preferably at least 67 years of age.

6. Subtilisin A hydrolysate of lysozyme for use in the method according to any one of the claims 1 - 5, wherein the subject is male, preferably a human male, more preferably a human male at least 60 years of age, preferably at least 65 years of age, more preferably at least 67 years of age, such as 60-105 years of age or 67-75 years of age.

7. Subtilisin A hydrolysate of lysozyme for use in the method according to any one of the claims 1-6, wherein at least 30% by weight of the subtilisin A hydrolysate of lysozyme are lysozyme peptides with a molecular weight of less than 0,5 kDa.

8. Subtilisin A hydrolysate of lysozyme for use in the method according to any one of the claims 1-7, wherein the degree of hydrolysis of the lysozyme is at least 12%, preferably 15% - 50%, more preferably 18% - 40%, most preferably 19% - 30%, such as 21%.

9. Subtilisin A hydrolysate of lysozyme for use in the method according to any one of the claims 1-8, wherein the daily dose of the subtilisin A hydrolysate of lysozyme is 0,5 gram - 20 gram per day, preferably 1 gram - 10 gram per day, more preferably 2 gram - 8 gram per day, such as 5 gram per day, preferably administered to the subject as a single daily dose.

10. A nutraceutical, a food product, a functional food, a food stuff, a food ingredient, a food supplement and/or a feed product, comprising a subtilisin A hydrolysate of lysozyme, for use in the method according to any one of the claims 1-9.

11. Non-therapeutic method of improving cognitive functioning in a subject, wherein said subject is a healthy human subject, the method comprising administering to the healthy human subject a composition comprising subtilisin A hydrolysate of lysozyme, wherein said improving cognitive function is any one or more of improving inhibitory control and improving control of impulsivity.

12. Non-therapeutic method of claim 11, wherein the subject is a human of at least 50 years of age, preferably at least 60 years of age, more preferably at least 60 years of age, most preferably at least 67 years of age.

13. Non-therapeutic method of any one of the claims 11-12, wherein at least 30% by weight of the subtilisin A hydrolysate of lysozyme are lysozyme peptides with a molecular weight of less than 0,5 kDa.

14. Non-therapeutic method of any one of the claims 11-13, wherein the degree of hydrolysis of the lysozyme is at least 12%, preferably 15% - 50%, more preferably 18% - 40%, most preferably 19% - 30%, such as 21%.

15. Non-therapeutic method of any one of the claims 11-14, wherein the daily dose of the subtilisin A hydrolysate of lysozyme is 0,5 gram - 20 gram per day, preferably 1 gram - 10 gram per day, more preferably 2 gram - 8 gram per day, such as 5 gram per day, preferably administered to the subject as a single daily dose.

16. Use of a nutraceutical, a food product, a functional food, a food stuff, a food ingredient, a food supplement, comprising a subtilisin A hydrolysate of lysozyme, in the non-therapeutic method according to any one of the claims 11-15.

## Patentansprüche

1. Subtilisin-A-Hydrolysat von Lysozym zur Verwendung in einem Verfahren zur Prophylaxe oder Behandlung von beeinträchtigter Hemmungskontrolle und/oder beeinträchtigter Impulskontrolle bei einem Patienten, der an einer oder mehreren der folgenden Erkrankungen leidet: neurodegenerativer Erkrankung, Alzheimer-Krankheit, Demenz, Parkinson-Krankheit, Zwangsstörung, Zwangsneurose, Tourette-Syndrom, Aufmerksamkeitsdefizit-Hyperaktivitätsstörung, psychiatrischer Störung, Trichotillomanie, Schizophrenie und einer Sucht nach einer Droge, einem Betäubungsmittel, Nikotin, Alkohol, Spielen und Glücksspielen leidet.

2. Subtilisin-A-Hydrolysat von Lysozym zur Verwendung in dem Verfahren nach Anspruch 1, wobei die Verwendung die Umkehrung zuvor verminderter kognitiver Funktionen des Patienten umfasst.

3. Subtilisin-A-Hydrolysat von Lysozym zur Verwendung in dem Verfahren nach Anspruch 1, wobei die Verwendung die Verbesserung oder Umkehrung zuvor verminderter kognitiver Funktionen umfasst.

4. Subtilisin-A-Hydrolysat von Lysozym zur Verwendung in dem Verfahren nach einem der Ansprüche 1 bis 3, wobei der Patient verminderte proaktive Hemmungskontrolle und/oder beeinträchtigte Impulskontrolle aufweist.

5. Subtilisin-A-Hydrolysat von Lysozym zur Verwendung in dem Verfahren nach einem der Ansprüche 1 bis 4, wobei der Patient ein Mensch im Alter von mindestens 50 Jahren, vorzugsweise mindestens 60 Jahren, noch bevorzugter mindestens 60 Jahren und am meisten bevorzugt mindestens 67 Jahren ist.

6. Subtilisin A-Hydrolysat von Lysozym zur Verwendung in dem Verfahren nach einem der Ansprüche 1 bis 5, wobei der Patient männlich ist, vorzugsweise ein männlicher Mensch, noch bevorzugter ein männlicher Mensch im Alter von mindestens 60 Jahren, vorzugsweise mindestens 65 Jahren, noch bevorzugter mindestens 67 Jahren, beispielsweise im Alter von 60 bis 105 Jahren oder 67 bis 75 Jahren.

7. Subtilisin A-Hydrolysat von Lysozym zur Verwendung in dem Verfahren nach einem der Ansprüche 1 bis 6, wobei mindestens 30 Gew.-% des Subtilisin A-Hydrolysats von Lysozym Lysozympeptide mit einem Molekulargewicht von weniger als 0,5 kDa sind.

8. Subtilisin-A-Hydrolysat von Lysozym zur Verwendung in dem Verfahren nach einem der Ansprüche 1 bis 7, wobei der Hydrolysegrad des Lysozyms mindestens 12 %, vorzugsweise 15 % bis 50 %, noch bevorzugter 18 % bis 40 %, am meisten bevorzugt 19 % bis 30 %, beispielsweise 21 %, beträgt.

9. Subtilisin-A-Hydrolysat von Lysozym zur Verwendung in dem Verfahren nach einem der Ansprüche 1 bis 8, wobei die Tagesdosis des Subtilisin-A-Hydrolysats von Lysozym 0,5 Gramm bis 20 Gramm pro Tag, vorzugsweise 1 Gramm bis 10 Gramm pro Tag, noch bevorzugter 2 Gramm - 8 Gramm pro Tag, beispielsweise 5 Gramm pro Tag, beträgt und vorzugsweise dem Patienten als einmalige Tagesdosis verabreicht wird.

10. Nutrazeutikum, ein Lebensmittelprodukt, ein funktionelles Lebensmittel, ein Nahrungsmittel, eine Lebensmittelzutat und/oder ein Futtermittelprodukt, das ein Subtilisin-A-Hydrolysat von Lysozym umfasst, zur Verwendung in dem Verfahren nach einem der Ansprüche 1 bis 9.

11. Nicht-therapeutisches Verfahren zur Verbesserung der kognitiven Funktionen bei einem Patienten, wobei der Patient ein gesunder Mensch ist, wobei das Verfahren die Verabreichung einer Zusammensetzung an den gesunden Menschen umfasst, die Subtilisin-A-Hydrolysat von Lysozym umfasst, wobei die Verbesserung der kognitiven Funktionen die Verbesserung der Hemmungskontrolle und/oder die Verbesserung der Impulskontrolle ist.

12. Nicht-therapeutisches Verfahren nach Anspruch 11, wobei der Patient ein Mensch im Alter von mindestens 50 Jahren, vorzugsweise mindestens 60 Jahren, noch bevorzugter mindestens 60 Jahren und am meisten bevorzugt mindestens 67 Jahren ist.

13. Nicht-therapeutisches Verfahren nach einem der Ansprüche 11 bis 12, wobei mindestens 30 Gew.-% des Subtilisin-A-Hydrolysats von Lysozym Lysozympeptide mit einem Molekulargewicht von weniger als 0,5 kDa sind.

14. Nicht-therapeutisches Verfahren nach einem der Ansprüche 11 bis 13, wobei der Hydrolysegrad des Lysozyms mindestens 12 %, vorzugsweise 15 % bis 50 %, noch bevorzugter 18 % bis 40 %, am meisten bevorzugt 19 % bis 30 %, beispielsweise 21 % beträgt.

15. Nicht-therapeutisches Verfahren nach einem der Ansprüche 11 bis 14, wobei die Tagesdosis des Subtilisin-A-Hydrolysats von Lysozym 0,5 Gramm bis 20 Gramm pro Tag, vorzugsweise 1 Gramm bis 10 Gramm pro Tag, noch bevorzugter 2 Gramm bis 8 Gramm pro Tag, beispielsweise 5 Gramm pro Tag, beträgt und vorzugsweise dem Patienten als einmalige Tagesdosis verabreicht wird.

16. Verwendung eines Nutrazeutikums, eines Lebensmittels, eines funktionellen Lebensmittels, eines Nahrungsmittels, einer Lebensmittelzutat, eines Nahrungsergänzungsmittels, das ein Subtilisin-A-Hydrolysat von Lysozym umfasst, in dem nicht-therapeutischen Verfahren nach einem der Ansprüche 11 bis 15.

## Revendications

1. Hydrolysat de lysozyme par la subtilisine A pour utilisation dans un procédé de prophylaxie ou de traitement d'un quelconque ou plusieurs parmi l'altération du contrôle inhibiteur et l'altération du contrôle de l'impulsivité chez un sujet souffrant d'une ou plusieurs affections parmi la maladie neurodégénérative, la maladie d'Alzheimer, la démence, la maladie de Parkinson, le trouble obsessionnel-compulsif, la névrose obsessionnelle, le syndrome de Gilles de La Tourette, le trouble déficitaire de l'attention avec hyperactivité, un trouble psychiatrique, la trichotillomanie, la schizophrénie et/ou la dépendance à l'un quelconque parmi une drogue, un stupéfiant, la nicotine, l'alcool, les jeux vidéo ou la ludopathie.

2. Hydrolysat de lysozyme par la subtilisine A pour utilisation dans le procédé de la revendication 1, où ladite utilisation comprend l'inversion du déclin cognitif antérieur dudit sujet.

3. Hydrolysat de lysozyme par la subtilisine A pour utilisation dans le procédé de la revendication 1, où ladite utilisation comprend l'amélioration ou l'inversion du déclin cognitif antérieur.

4. Hydrolysat de lysozyme par la subtilisine A pour utilisation dans le procédé de l'une quelconque des revendications 1 à 3, où ledit sujet présente au moins un ou plusieurs parmi un dysfonctionnement du contrôle inhibiteur proactif et une altération du contrôle de l'impulsivité.

5. Hydrolysat de lysozyme par la subtilisine A pour utilisation dans le procédé de l'une quelconque des revendications 1 à 4, où le sujet est un être humain âgé d'au moins 50 ans, de préférence d'au moins 60 ans, plus préférablement d'au moins 60 ans, et de manière encore plus préférable d'au moins 67 ans.

6. Hydrolysat de lysozyme par la subtilisine A pour utilisation dans le procédé selon l'une quelconque des revendications 1 à 5, où le sujet est un sujet masculin, de préférence un homme humain, plus préférablement un homme humain âgé d'au moins 60 ans, de préférence d'au moins 65 ans, de manière encore plus préférable d'au moins 67 ans, par exemple de 60 à 105 ans ou de 67 à 75 ans.

7. Hydrolysat de lysozyme par la subtilisine A pour utilisation dans le procédé selon l'une quelconque des revendications 1 à 6, où au moins 30 % en poids de l'hydrolysat de lysozyme par la subtilisine A sont des peptides de lysozyme ayant un poids moléculaire inférieur à 0,5 kDa.

8. Hydrolysat de lysozyme par la subtilisine A pour utilisation dans le procédé selon l'une quelconque des revendications 1 à 7, où le degré d'hydrolyse du lysozyme est d'au moins 12 %, de préférence de 15 % à 50 %, plus préférablement de 18 % à 40 %, et de manière encore plus préférable de 19 % à 30 %, par exemple 21 %.

9. Hydrolysat de lysozyme par la subtilisine A pour utilisation dans le procédé selon l'une quelconque des revendications 1 à 8, où la dose quotidienne de l'hydrolysat de lysozyme par la subtilisine A est de 0,5 g à 20 g par jour, de préférence de 1 g à 10 g par jour, plus préférablement de 2 g à 8 g par jour, par exemple 5 g par jour, de préférence administrée au sujet en une seule dose quotidienne.

10. Nutraceutique, produit alimentaire, aliment fonctionnel, denrée alimentaire, ingrédient alimentaire, complément alimentaire et/ou aliment pour animaux, comprenant un hydrolysat de lysozyme par la subtilisine A, pour utilisation dans le procédé selon l'une quelconque des revendications 1 à 9.

11. Procédé non thérapeutique d'amélioration des fonctions cognitives chez un sujet, où ledit sujet est un sujet humain sain, le procédé comprenant l'administration au sujet humain sain d'une composition comprenant un hydrolysat de lysozyme par la subtilisine A, où ladite amélioration des fonctions cognitives est l'une ou plusieurs parmi l'amélioration du contrôle inhibiteur et l'amélioration du contrôle de l'impulsivité.

12. Procédé non thérapeutique selon la revendication 11, où le sujet est un être humain âgé d'au moins 50 ans, de préférence d'au moins 60 ans, plus préférablement d'au moins 60 ans, et de manière encore plus préférable d'au moins 67 ans.

13. Procédé non thérapeutique selon l'une quelconque des revendications 11 à 12, où au moins 30 % en poids de l'hydrolysat de lysozyme par la subtilisine A sont des peptides de lysozyme ayant un poids moléculaire inférieur à 0,5 kDa.

14. Procédé non thérapeutique selon l'une quelconque des revendications 11 à 13, où le degré d'hydrolyse du lysozyme est d'au moins 12 %, de préférence de 15 % à 50 %, plus préférablement de 18 % à 40 %, et de manière encore plus préférable de 19 % à 30 %, par exemple 21 %.

15. Procédé non thérapeutique selon l'une quelconque des revendications 11 à 14, où la dose quotidienne de l'hydrolysat de lysozyme par la subtilisine A est de 0,5 g à 20 g par jour, de préférence de 1 g à 10 g par jour, plus préférablement de 2 g à 8 g pajour, par exemple 5 g par jour, de préférence administrée au sujet en une seule dose quotidienne.

16. Utilisation d'un nutraceutique, d'un produit alimentaire, d'un aliment fonctionnel, d'une denrée alimentaire, d'un ingrédient alimentaire ou d'un complément alimentaire, comprenant un hydrolysat de lysozyme par la subtilisine A, selon le procédé non thérapeutique selon l'une quelconque des revendications 11 à 15.
